Europäisches Patentamt
European Patent Office
Office européen des brevets

(19)

(11) Publication number: 0 323 864 A2

# (12) EUROPEAN PATENT APPLICATION

(21) Application number: 89200001.9

(22) Date of filing: 02.01.89

(51) Int. Cl.[4]: C 07 D 453/02
C 07 D 471/08,
C 07 D 413/04,
C 07 D 453/06, A 61 K 31/44,
C 07 D 309/12,
C 07 D 211/78,
C 07 D 413/14,
C 07 D 207/16, C 07 D 405/12

(30) Priority: 08.01.88 GB 8800394
08.06.88 GB 8813513
24.10.88 GB 8824898

(43) Date of publication of application:
12.07.89 Bulletin 89/28

(84) Designated Contracting States:
AT BE CH DE ES FR GB GR IT LI LU NL SE

(71) Applicant: MERCK SHARP & DOHME LTD.
Hertford Road
Hoddesdon Hertfordshire EN11 9BU (GB)

(72) Inventor: Baker, Raymond
Bulls Cottage Green Tye
Much Hadham Hertfordshire (GB)

Merchant, Kevin J.
29 Red Lion Court Dane Street
Bishops Stortford Hertfordshire (GB)

Saunders, John
13 The Ridings Thorley Park
Bishops Stortford Hertfordshire (GB)

Street, Leslie J.
99 Spruce Hill Harlow
Essex (GB)

(74) Representative: Hesketh, Alan, Dr.
European Patent Department Merck & Co., Inc. Terlings
Park Eastwick Road
Harlow Essex, CM20 2QR (GB)

Claims for the following Contracting States: ES + GR.

## (54) Lipophilic oxadiazoles.

(57) A class of oxadiazoles, substituted on one of the ring carbon atoms by a non-aromatic azacyclic or azabicyclic ring system, and on the other by an optionally substituted saturated hyrocarbon group having at least three carbon atoms, or unsaturated hydrocarbon group having at least six carbon atoms; have particularly advantageous selective properties for use in the treatment and/or prevention of neurodegenerative diseases.

EP 0 323 864 A2

**Description**

## LIPOPHILIC OXADIAZOLES

The present invention relates to a class of substituted oxadiazole compounds which stimulate central muscarinic acetylcholine receptors and therefore are useful in the treatment of neurological and mental illnesses whose clinical manifestations are due to cholinergic deficiency. Such diseases include presenile and senile dementia (also known as Alzheimer's disease and senile dementia of the Alzheimer type respectively), Huntington's chorea, tardive dyskinesia, hyperkinesia, mania and Tourette Syndrome. Alzheimer's disease, the most common dementing illness, is a slowly progressive neurological disorder characterised by marked deficits in cognitive functions including memory, attention, language and visual perception capabilities.

Published European Patent Application No. 239309 discloses a class of oxadiazole compounds having a substituent of low lipophilicity, which are useful in the treatment of nuerodegenerative disorders. It has now been found that a further class of oxadiazoles having different substituents also stimulate cholinergic transmission, but additionally have particularly advantageous selective properties for use in the treatment of neurodegenerative diseases. The compounds of the present invention stimulate muscarinic cholinergic transmission in the cortex and thereby reverse the cholinergic deficiency, as do the class of compounds of EP-A-0239309, but achieve this by a mechanism selective for the cortex. That is, the present compounds do not stimulate cholinergic transmission in peripheral tissues such as heart, smooth muscle and glandular tissues. The compounds of this invention therefore have a more selective action, leading to fewer undesirable side-effects such as cardiovascular complications, diarrhoea, and salivation.

Accordingly the present invention provides a compound of formula I, or a salt or prodrug thereof:

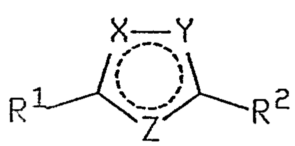

(I)

wherein one of X, Y and Z is an oxygen atom and the other two are nitrogen atoms, and the dotted circle represents aromaticity (two double bonds) thus forming a 1,3,4-oxadiazole or 1,2,4-oxadiazole nucleus; $R^1$ represents a non-aromatic azacyclic or azabicyclic ring system; and $R^2$ represents an optionally substituted saturated hydrocarbon group having at least three carbon atoms, or unsaturated hydrocarbon group having at least 6 carbon atoms.

The azacyclic or azabicyclic ring system is a non-aromatic ring system containing one nitrogen atom as the sole heteroatom. Suitably the ring system contains from 4 to 10 ring atoms, preferably from 5 to 8 ring atoms. The bicyclic systems may be fused, spiro or bridged. Examples of suitable ring systems include the following:

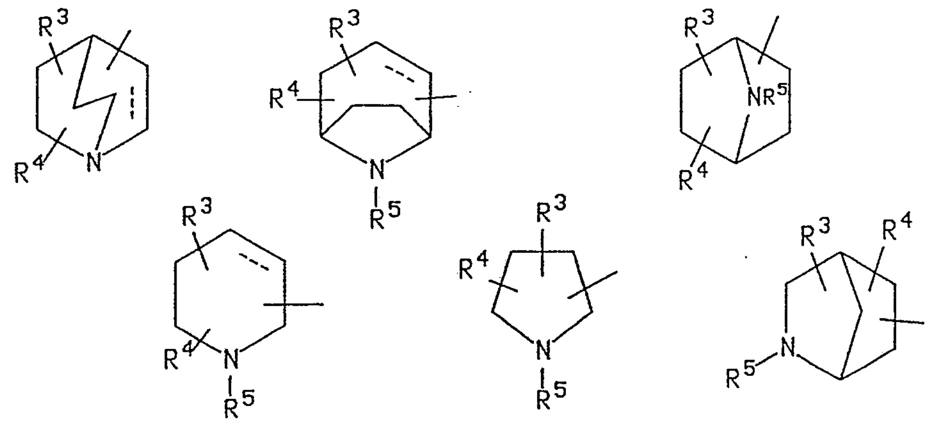

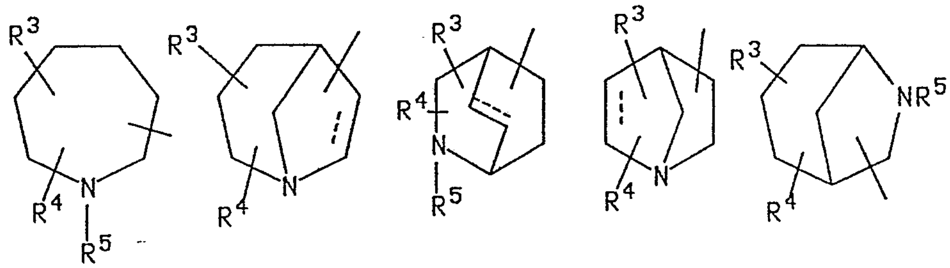

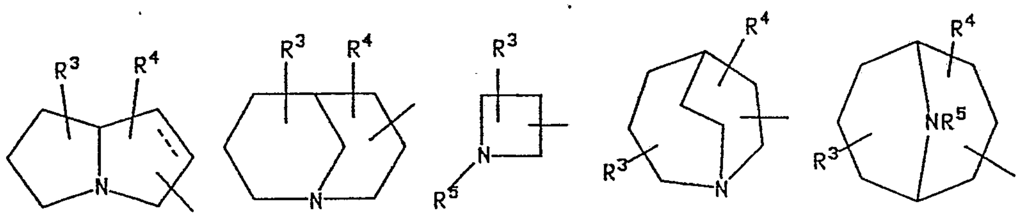

wherein the broken line represents an optional chemical bond;
the substituents $R^3$ and $R^4$ may be present at any position, including the point of attachment to the oxadiazole ring, and independently represent hydrogen, $C_{1-4}$ alkyl, halo, $C_{1-4}$ alkoxy, hydroxy-methyl, amino, hydroxy or carboxy; or $R^3$ and $R^4$ together represent carbonyl; and
$R^5$ represents hydrogen or $C_{1-4}$ alkyl.

It will be appreciated that the nitrogen atom in the azacyclic or azabicyclic ring will carry a lone pair of electrons.

Preferably the oxadiazole ring is a 1,2,4-oxadiazole.

Suitably the group $R^3$ is hydrogen or methyl; and $R^4$ is hydrogen, $C_{1-4}$ alkoxy, $C_{1-4}$ alkyl or hydroxy, preferably methoxy, methyl or hydroxy. Preferably one or both of $R^3$ and $R^4$ is hydrogen.

Preferably the group $R^5$ represents hydrogen or methyl.

Suitably the azacyclic or azabicyclic ring system is pyrrolidine, piperidine, tetrahydropyridine, 1- or 2-azanorbornane, quinuclidine, isoquinuclidine or 2-azabicyclo[2.2.2]octene, any of which may be optionally substituted with methyl or hydroxy.

The hydrocarbon group $R^2$ may be $C_{3-15}$ alkyl, $C_{6-15}$ alkenyl, $C_{6-15}$ alkynyl, aryl, aralkyl, aralkenyl or heteorarylalkyl. The alkyl, alkenyl or alkynyl groups may be straight, branched or cyclic groups. Suitably the alkyl group comprises from 3 to 6 carbon atoms. The hydrocarbon group may carry one or more substituents. Suitable substituent groups for the hydrocarbon group $R^2$ include halogen, $-OR^6$, $-CF_3$, $-NR^6R^7$, $-NO_2$, methyl, ethyl, vinyl, optionally substituted aryl, optionally substituted heteroaryl, keto, $-SR^6$, $-SOR^6$, $-SO_2R^6$, $-CO_2R^6$ and $-CONR^6R^7$; wherein $R^6$ and $R^7$ independently represent hydrogen, $C_{1-6}$ alkyl or - $COCH_3$.

Suitable aryl groups are phenyl and naphthyl. Suitable heteroaryl groups include furyl, thiophenyl, pyridyl and pyrrolyl.

Preferred substituents for the hydrocarbon group $R^2$ include methyl, vinyl, phenyl, naphthyl, pyridyl, methylcarbonyloxy, hydroxy and methoxy.

Substituents most suitable for the aryl and heteroaryl groups include fluoro, chloro, bromo, methoxy, $C_{1-6}$ alkyl, methoxycarbonyl, trifluoromethyl, nitro and $-NR^6R^7$.

Particular values of the group $R^2$ include phenyl $(C_{1-3})$alkyl, $C_{3-6}$ cycloalkyl, $C_{3-6}$ alkyl and adamantyl.

One class of groups $R^2$ comprises n-propyl, isopropyl, cyclopropyl, n-butyl, benzyl, phenylethenyl or

pyridylmethyl, optionally substituted by one or more substituents selected from methyl, ethyl, vinyl, phenyl, napthyl, pyridyl, acetoxy, keto, fluoro, hydroxy and methoxy. A preferred group $R^2$ has the structure:

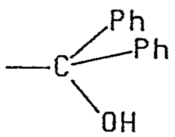

One sub-class of compounds within the scope of the present invention is represented by formula II:

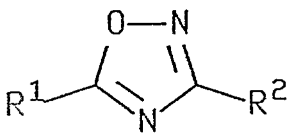

(II)

wherein $R^1$ and $R^2$ are as defined above; in particular wherein $R^1$ represents pyrrolidine, quinuclidine, tetrahydropyridine, piperidine, dehydrotropane, pyrrolizidine, azanorbornane, isoquinuclidine, or 2-azabicyclo[2.2.2]octene, any of which groups $R^1$ may be optionally substituted with $C_{1-3}$ alkyl, or hydroxy; and $R^2$ represents n-propyl, isopropyl, cyclopropyl, n-butyl, 4-chlorophenyl, benzyl, 1-phenylethyl, adamantyl, 1-hydroxy-1-phenylmethyl, 1-acetoxy-1-phenylmethyl, benzoyl, 1-hydroxy-1-phenylethyl, 1,1-diphenyl-1-hydroxymethyl, 2-methoxy-1-phenylethenyl, 1-hydroxy-1-phenylpropyl, 1,1-diphenylmethyl, 1-hydroxy-1-phenylprop-2-enyl, 1-hydroxy-1-(2-naphthyl)-1-phenylmethyl, 1-(4-fluorophenyl)-1-hydroxy-1-phenylmethyl, 1-hydroxy-1-(4-methoxyphenyl)-1-phenylmethyl, 1-hydroxy-1-(pyrid-2-yl)methyl or 1-hydroxy-1-phenyl-1-(pyrid-2-yl)methyl.

Specific compounds of the present invention include:
3-[5-(3-benzyl-1,2,4-oxadiazol)yl]quinuclidine;
3-[5-(3-cyclopropyl-1,2,4-oxadiazol)yl]quinuclidine;
3-[5-(3-(1-phenyl ethyl-1,2,4-oxadiazol)yl]quinuclidine;
3-[5-(3-(1-adamantyl)-1,2,4-oxadiazol)yl]quinuclidine; 3-[5-(3-cyclopropyl-1,2,4-oxadiazol)yl]-1-azabicyclo-[2.2.1]heptane;
3-[5-(3-cyclopropyl-1,2,4-oxadiazol)yl]-1,2,5,6-tetrahydropyridine;
3-[5-(3-benzyl-1,2,4-oxadiazol)yl]-1,2,5,6-tetrahydropyridine;
3-[5-(3-isopropyl-1,2,4-oxadiazol)yl]-1,2,5,6-tetrahydropyridine;
3-cyclopropyl-5-(5-ethyl-1,2,5,6-tetrahydropyrid-3-yl)-1,2,4-oxadiazole;
3-isopropyl-5-(5-ethyl-1,2,5,6-tetrahydropyrid-3-yl)-1,2,4-oxadiazole;
3-cyclopropyl-5-(5-methyl-1,2,5,6-tetrahydropyrid-3-yl)-1,2,4-oxadiazole;
3-isopropyl-5-(5-methyl-1,2,5,6-tetrahydropyrid-3-yl)-1,2,4-oxadiazole;
3-[3-(1-hydroxy-1-phenylmethyl)-1,2,4-oxadiazol-5-yl]quinuclidine;
3-[3-(1-acetoxy-1-phenylmethyl)-1,2,4-oxadiazol-5-yl]quinuclidine;
3-(3-benzoyl-1,2,4-oxadiazol-5-yl)quinuclidine; 3-[3-(1-hydroxy-1-phenylethyl)-1,2,4-oxadiazol-5-yl]quinuclidine;
3-[3-(1,1-diphenyl-1-hydroxymethyl)-1,2,4-oxadiazol-5-yl]quinuclidine;
3-[3-(2-methoxy-1-phenylethenyl)-1,2,4-oxadiazol-5-yl]quinuclidine;
3-[5-(1-hydroxy-1-phenylmethyl)-1,2,4-oxadiazol-3-yl]quinuclidine;
3-[3-(1,1-diphenyl-1-hydroxymethyl)-1,2,4-oxadiazol-5-yl]-1,2,5,6-tetrahydropyridine;
3-[3-(1-hydroxy-1-phenylmethyl)-1,2,4-oxadiazol-5-yl]-1,2,5,6-tetrahydropyridine;
3-[3-(4-chlorophenyl)-1,2,4-oxadiazol-5-yl]-1,2,5,6-tetrahydropyridine;
5-hydroxy-3-(3-isopropyl-1,2,4-oxadiazol-5-yl)-1-azabicyclo[2.2.1]heptane;
3-(3-cyclopropyl-1,2,4-oxadiazol-5-yl)-5-hydroxy-1-azabicyclo[2.2.1]heptane;
5-hydroxy-3-(3-n-propyl-1,2,4-oxadiazol-5-yl)-1-azabicyclo[2.2.1]heptane;
3-(3-n-propyl-1,2,4-oxadiazol-5-yl)-1-azabicyclo[2.2.1]heptane;
3-(3-cyclopropyl-1,2,4-oxadiazol-5-yl)-1-azabicyclo[2.2.1]heptane;
3-(3-isopropyl-1,2,4-oxadiazol-5-yl)-1-azabicyclo[2.2.1]heptane;
3-[3-(1-phenylethyl)-1,2,4-oxadiazol-5-yl]-1-azabicyclo[2.2.1]heptane;
3-[3-(1-hydroxy-1-phenylpropyl)-1,2,4-oxadiazol-5-yl]quinuclidine;
3-[3-(1,1-diphenylmethyl)-1,2,4-oxadiazol-5-yl]quinuclidine;

3-[3-(1-hydroxy-1-phenylprop-2-enyl)-1,2,4-oxadiazol-5-yl]quinuclidine;
3-[3-(1-hydroxy-1-(2-naphthyl)-1-phenylmethyl)-1,2,4-oxadiazol-5-yl]quinuclidine;
3-[3-(1-(4-fluorophenyl)-1-hydroxy-1-phenylmethyl)-1,2,4-oxadiazol-5-yl]quinuclidine;
3-[3-(1-hydroxy-1-(4-methoxyphenyl)-1-phenylmethyl)-1,2,4-oxadiazol-5-yl]quinuclidine;
3-[3-(1,1-diphenyl-1-hydroxymethyl)-1,2,4-oxadiazol-5-yl]-1-azabicyclo[2.2.1]heptane;
3-[3-(1-hydroxy-1-(pyrid-2-yl)methyl-1,2,4-oxadiazol-5-yl]quinuclidine;
3-[3-(1-hydroxy-1-phenyl-1-(pyrid-2-yl)methyl-1,2,4-oxadiazol-5-yl]quinuclidine;
6-(3-cyclopropyl-1,2,4-oxadiazol-5-yl)-2-azabicyclo[2.2.2]octane;
6-(3-isopropyl-1,2,4-oxadiazol-5-yl)-2-azabicyclo[2.2.2]octane;
6-(3-cyclopropyl-1,2,4-oxadiazol-5-yl)-2-methyl-2-azabicyclo[2.2.2]octane;
6-(3-cyclopropyl-1,2,4-oxadiazol-5-yl)-2-azabicyclo[2.2.2]oct-7-ene;
3-(3-n-butyl-1,2,4-oxadiazol-5-yl)-1-azabicyclo[2.2.1]heptane;
and salts and prodrugs thereof.

European Specification 259621, filed 7 August 1987, discloses compounds which overlap with the present invention when formula I above represents a 1,2,4-oxadiazole; $R^1$ represents:

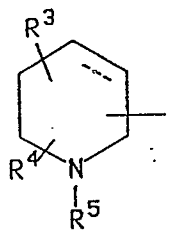

wherein $R^3$, $R^4$ and $R^5$ are as defined above; and $R^2$ represents straight or branched chain $C_{3-8}$ alkyl, cyclopropyl or phenyl.

Most of the compounds of this invention have at least one asymmetric centre and often more than one; and can therefore exist as both enantiomers and diastereoisomers. In particular, those compounds possessing an unsymmetrical azabicyclic ring system may exist as exo and endo isomers. It is to be understood that the invention covers all such isomers and mixtures thereof.

Also included within the scope of the present invention are salts of the novel compounds. It will be appreciated that salts of the compounds for use in medicine will be non-toxic pharmaceutically acceptable salts. Other salts may, however, be useful in the preparation of the compounds of the invention or their non-toxic pharmaceutically acceptable salts. Acid addition salts, for example, may be formed by mixing a solution of the compound with a solution of a pharmaceutically acceptable non-toxic acid such as hydrochloric acid, fumaric acid, maleic acid, succinic acid, acetic acid, citric acid, tartaric acid, carbonic acid or phosphoric acid. Where the novel compound carries a carboxylic acid group the invention also contemplates salts thereof, preferably non-toxic pharmaceutically acceptable salts thereof, such as the sodium, potassium and calcium salts thereof.

Salts of amine groups may also comprise the quaternary ammonium salts in which the amino nitrogen atom carries an alkyl, alkenyl, alkynyl or aralkyl group. Such quaternary ammonium derivatives penetrate poorly into the central nervous system and are therefore useful as peripherally selective muscarinic agents, useful for example as antispasmodic agents, agents to reduce gastric acid secretion, agents to block the muscarinic actions of acetylcholinesterase inhibitors in the treatment of myasthenia gravis and as agents to co-administer with muscarinic agonists in Alzheimer's disease.

It is believed that the selective enhancement of central cholinergic transmission demonstrated by the compounds of this invention is achieved by one of two alternative mechanisms. One possible mechanism is potentiation of presynaptic acetylcholine release specifically in the cortex. The compounds antagonise presynaptic receptors which are responsible for regulating the release of acetylcholine. Because such compounds fail to stimulate the receptors located in peripheral tissues, side-effects in those tissues are reduced.

Another possible mechanism involves the direct selective activation of one or more of the sub-types of muscarinic receptor located in the cortex without affecting those sub-types present in peripheral tissues leading again to compounds with fewer undesirable side-effects.

The compounds according to the invention have been found to demonstrate an affinity for the muscarinic receptor, and their tissue selectivity was assessed in three functional models, namely (i) rat superior cervical ganglion; (ii) rat atrium; and (iii) guinea pig ileum. These functional models have been described in J. Med. Chem., 1987, 30, 969. Certain of the compounds demonstrated a selective stimulant action at the ganglionic muscarinic receptor relative to the atrial and ileal receptors. Other compounds, meanwhile, appeared to antagonise the atrial and ileal sites selectively, and may therefore act by stimulating acetylcholine release in the cortex.

The method of treatment of this invention includes a method of treating Alzheimer's disease, senile dementia of the Alzheimer type, Huntington's chorea, tardive dyskinesia, hyperkinesia, mania or Tourette syndrome by the administration to a patient in need of such treatment of an effective amount of one or more of the novel compounds.

This invention therefore also provides a pharmaceutical composition comprising a compound of the invention and a pharmaceutically acceptable carrier.

The compounds of the invention can be administered orally, parenterally or rectally at a daily dose of about 0.01 to 10 mg/kg of body weight, preferably about 0.1 to 1 mg/kg, and may be administered on a regimen of 1 to 4 times a day.

The pharmaceutical formulations of this invention preferably are in unit dosage forms such as tablets, pills, capsules, powders, granules, sterile parenteral solutions or suspensions, or suppositories for oral, parenteral or rectal administration. For preparing solid compositions such as tablets, the principal active ingredient is mixed with a pharmaceutical carrier, e.g. conventional tabletting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids or mixtures of polymeric acids with such materials as shellac, cetyl alcohol and cellulose acetate.

The liquid forms in which the novel compositions of the present invention may be incorporated for administration orally or by injection include aqueous solutions, suitably flavoured syrups and flavoured emulsions with edible oils such as cottonseed oil, sesame oil, coconut oil and peanut oil, as well as elixirs and similar pharmaceutical vehicles. Suitable dispersing or suspending agents for aqueous suspension include synthetic and natural gums such as tragacanth, acacia, alginate, dextran, sodium carboxymethylcellulose, methylcellulose, polyvinyl-pyrrolidone and gelatin.

The compounds of this invention may be prepared by a process which comprises reacting a reactive derivative of a carboxylic acid of formula $R^a$-$CO_2H$ with a compound either of formula IIIA or of formula IIIB, or a salt thereof:

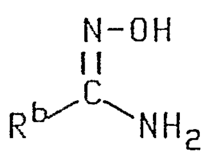

(IIIA)

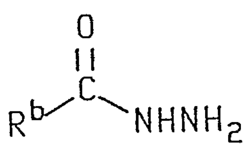

(IIIB)

wherein one of $R^a$ and $R^b$ is a non-aromatic azacyclic or azabicyclic ring, and the other is an optionally substituted saturated hydrocarbon group having at least three carbon atoms, or unsaturated hydrocarbon group having at least 6 carbon atoms.

Suitable reactive derivatives of the acid $R^a$-$CO_2H$ include esters, for example $C_{1-4}$ alkyl esters; thioesters, for example pyridylthioesters; acid anhydrides, for example $(R^aCO)_2O$; acid halides, for example acid chlorides; orthoesters; and primary, secondary and tertiary amides.

When the compound of formula IIIA is employed the product of the reaction is a 1,2,4-oxadiazole. It will be appreciated that the compound IIIA can also be considered as the alternative tautomeric form:

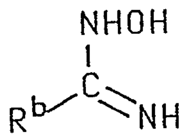

A 3-substituted-1,2,4-oxadiazol-5-yl compound is produced if $R^a$ represents the azacyclic or azabicyclic group and $R^b$ in formula IIIA represents the hydrocarbon substituent. In this case, a preferred reactive derivative of the acid $R^aCO_2H$ is a $C_{1-4}$ alkyl ester. The reaction is conveniently carried out in tetrahydrofuran, dimethylformamide or a lower alkanol such as ethanol, propanol or isopropanol at about 20° to 100°C for about 1 to 6 hours.

A 5-substituted-1,2,4-oxadiazol-3-yl compound is produced by the process of this invention when $R^a$ represents the hydrocarbon substituent and $R^b$ represents the azacyclic or azabicyclic group. For this reaction a suitable reactive derivative is the acid chloride or the acid anhydride $(R^aCO)_2$. The reaction may be carried out by treating compound IIIA, in the cold, e.g. from about -5° to +10°C, with the reactive derivative, followed by heating at about 80°C to 120°C for about 1 to 6 hours.

When the compound of formula IIIB is employed, the product of the process of this invention is a 1,3,4-oxadiazole. In this case, a preferred reactive derivative of the acid $R^aCO_2H$ is an orthoester of formula $R^aC(OR^8)_3$ where $R^8$ represents $C_{1-3}$ alkyl. The process is conveniently effected by heating the hydrazide IIIB with the orthoester in a solvent such as methanol at reflux temperature for about 2 to 8 hours. An intermediate of formula $R^b.CO.NH.N=C(R^a)OR^8$ may be isolated by evaporation of the solvent. The intermediate is then treated with a strong base such as potassium t-butoxide or 1,8-diazabicyclo[5.4.0]undec-7-ene, in butanol for about 10 to 24 hours at about 90° to 150°C.

After the above process is complete, one substituent on the hydrocarbon group, or the azacyclic or azabicyclic ring, can be converted to another. For example an amino group may be converted to chloro, or hydrazo, $-NHNH_2$, via the intermediacy of diazonium, $-N_2^+$. Similarly, a chloro substituent may be converted to methoxy by reaction with a nucleophile such as methoxide; alkoxycarbonyl groups may be converted, via carboxy, to an amino substituent, $-NH_2$; and keto compounds may be converted, by reaction with an organometallic reagent such as a Grignard reagent, to tertiary alcohol derivatives.

In any of the above reactions it may be necessary and/or desirable to protect any sensitive groups in the compounds. For example, if $R^a$ and/or $R^b$ include amino, carboxy, hydroxy or thiol groups, these may be protected in conventional manner. Thus, suitable protecting groups for hydroxy groups include silyl groups such as trimethylsilyl or t-butyldi-methylsilyl, and etherifying groups such as tetrahydropyranyl; and for amino groups include benzyloxycarbonyl and t-butyloxycarbonyl. Carboxy groups are preferably protected in a reduced form such as in the form of their corresponding protected alcohols, which may be subsequently oxidised to give the desired carboxy group. Thiol groups may be protected by disulphide formation, either with the thiol itself or with another thiol to form a mixed disulphide. The protecting groups may be removed at any convenient stage in the synthesis of the desired compound according to conventional techniques.

The following Examples illustrate the preparation of compounds according to the invention. The compounds demonstrate an affinity for the muscarinic receptor, having an $IC_{50}$ (concentration required to displace 50% of specific [$^3$H]-N-methylscopolamine binding from rat cortical membrane preparation) better than 10 μM.

The compounds of each of the accompanying Examples display penetrability into the central nervous system, as assessed by a measurable displacement of radioligand binding using standard "ex-vivo" binding techniques (Ref: J. Neurosurg., 1985, 63, 589-592).

## EXAMPLE 1

3[5-(3-Benzyl-1,2,4-oxadiazol)yl]quinuclidine hemi-oxalate

Sodium hydride (1.8g of an 80% dispersion in oil, 60mmol) was added to a stirred solution of phenyl-acetamide oxime (9.0g, 60mmol) in anhydrous tetrahydrofuran (200ml) and the reaction mixture heated at reflux for 0.5 hours. 3-Methoxycarbonyl quinuclidine (6.1g, 36mmol) was added and the reaction heated under reflux for 2 hours. Water (50ml) was added, after allowing the reaction to cool to room temperature, and the product extracted into dichloromethane (4 x 150ml). The combined extracts were dried (sodium sulphate), the solvent removed under reduced pressure, and the residue purified by chromatography on alumina eluting with ethyl acetate to give the title compound free base (2.8g) which was crystallised from ethanol/ether as the hemi-oxalate salt, mp 163-165°C; (Found: C, 65.15, H, 6.38, N, 13.45. $C_6H_{19}N_3O$ (COOH) requires C, 64.95, H, 6.41, N, 13.36%); m/e 270 ($M^+$ of free base); δ ($D_2O$, 360MHz) 1.60-2.00 and 2.09-2.16 (each 2H, each m, 5-$CH_2$ and 8-$CH_2$), 2.59-2.62 (1H, m, 4-CH) 3.32-3.49 (4H, m, 6-$CH_2$ and 7-$CH_2$); 3.75-3.90 (3H, m, 3-CH and 2-$CH_2$), 4.17 (2H, s, $CH_2$Ph) and 7.35-7.44 (5H, m, Ph).

The following examples were prepared by the same method detailed in Example 1, but using the appropriate

ester and amide oxime.

EXAMPLE 2

3-[5-(3-Cyclopropyl-1,2,4-oxadiazol)yl]quinuclidine hydrochloride

This was prepared from cyclopropyl carboxamide oxime and 3-methoxycarbonyl quinuclidine and recrystallised from isopropyl alcohol/ether, mp 175-177°C; (Found: C, 56.34; H, 7.12; N, 16.26. $C_{12}H_{17}N_3O$. HCl requires C, 56.36, H, 7.05; N, 16.44%); m/e 219 ($M^+$ of free base); δ ($CDCl_3$, 360MHz) 0.95-1.05 (4H, m, 2x cyclopropyl $CH_2$), 1.38-1.42 (1H, m, cyclopropyl CH), 1.58-1.70 (3H, m, 5-$CH_2$ and one of 8-$CH_2$), 2.00-2.30 (2H, m, 4-CH and one of 8-$CH_2$), 2.80-3.40 (7H, m, 6-$CH_2$, 7-$CH_2$, 2-$CH_2$ and 3-CH).

EXAMPLE 3

3-[5-(3-(1-Phenyl)ethyl,1,2,4-oxadiazol)yl]quinuclidine hydrogen oxalate

Prepared from 3-methoxycarbonyl quinuclidine with 2-phenyl propionamide oxime and crystallised from diethyl ether, mp 102-103°C; (Found: C, 60.9; H, 6.0; N, 11.0. $C_{17}H_{21}N_3O.(COOH)_2$ requires C, 61.1, M, 6.2; N, 11.3%); m/e 283 ($M^+$ of free base); δ ($D_2O$, 360MHz) 1.67 (3H, d, J = 7Hz, $CH_3$). 1.66-1.79 (1H, m) with 1.83-1.96 (1H, m) and 2.05-2.21 (2H, m) for 5-$CH_2$ and 8-$CH_2$, 2.56-2.62 (1H, m, 4-CH), 3.28-3.48 (4H, m, 6-$CH_2$ and 7-$CH_2$), 3.74-3.88 (3H, m, 2-$CH_2$ and 3-CH), 4.37 (1H, q, J = 7Hz, C<u>H</u>$CH_3$) and 7.30-7.42 (5H, m, Ph).

EXAMPLE 4

3-[5-(3-(1-Adamantanyl)-1,2,4-oxadiazol)yl]quinuclidine

Prepared from 3-methoxycarbonyl quinuclidine with 1-adamantane carboxamide oxime and crystallised from ethanol, mp 205-206°C; (Found: C, 71.6; H, 8.7.; N, 13.3. $C_{19}H_{27}N_3O.0.25H_2O$ requires C, 71.8; H, 8.7; N, 13.2%); m/e 313 ($[M+1]^+$ of free base); δ ($CDCl_3$, 360MHz) 1.37-1.48 (1H, m) with 1.56-1.76 (3H, m) (5-$CH_2$ and 8-$CH_2$), 1.79 (6H, bs, 3x adamantyl $CH_2$), 2.02 (6H, bs, 3x adamantyl $CH_2$), 2.08 (3H, bs, 3x adamantyl CH), 2.20-2.25 (1H, m, 4-CH), 2.82-3.07 (4H, m, 6-$CH_2$ and 7-$CH_2$), 3.14-3.34 (3H, m, 2-$CH_2$ and 3-CH).

EXAMPLE 5

3-[5-(3-Cyclopropyl-1,2,4-oxadiazol)yl]-1-azabicyclo[2.2.1]heptane hydrochloride

Prepared from 3-methoxycarbonyl-1-azabicyclo[2.2.1]heptane and cyclopropyl carboxamide oxime and crystallised from isopropyl alcohol/ether, mp 157-159°C; (Found: C, 54.44; H, 6.63; N, 17.24. $C_{11}H_{15}N_3O$. HCl requires C, 54.66; H, 6.67; N, 17.38%); m/e 205 ($M^+$ of free base); δ ($D_2O$, 360MHz) 0.96-1.01 (2H, m, cyclopropyl $CH_2$), 1.12-1.19 (2H, m, cyclopropyl $CH_2$), 1.98-2.32 (3H, m, cyclopropyl CH and 5-$CH_2$) and 3.29-3.86 (8H, m, remainder of bicycloheptane H).

EXAMPLE 6

3-[5-(3-Cyclopropyl-1,2,4-oxadiazol)yl]-1,2,5,6-tetrahydropyridine hydrochloride

This was prepared from cyclopropyl carboxamide oxime and 3-methoxycarbonyl-1-vinyloxycarbonyl-1,2,5,6-tetrahydropyridine (R.A. Olofson et al, Tet. Letts. 1977, 1567) and recrystallised from isopropyl alcohol/diethyl ether, mp 177-178°C; (Found: C, 52.82; H, 6.27; N, 17.92. $C_{10}H_{13}N_3O$. HCl requires C, 52.75; H, 6.20; N, 18.45%); m/e 190 ($(M-H)^+$ of free base); δ ($D_2O$, 360MHz) 0.98-1.03 and 1.13-1.19 (each 2H, each m, 2 x cyclopropyl $CH_2$), 2.09-2.17 (1H, m, cyclopropyl CH), 2.71-2.77 (2H, m, 5-$CH_2$), 3.46 (2H, dd, J = 6Hz, 6-$CH_2$), 4.12-4.14 (2H, m, 2-$CH_2$), 7.27-7.30 (1H, m, 4-CH).

EXAMPLE 7

3-[5-(3-Benzyl-1,2,4-oxadiazol)yl]-1,2,5,6-tetrahydropyridine hydrochloride

This was prepared from phenylacetamide oxime and 3-methoxycarbonyl-1-vinyloxy-carbonyl-1,2,5,6-tetrahydropyridine and recrystallised from methanol/diethyl ether, mp 154-158°C, Rf = 0.63 in dichloro-methane/methanol (20:1) on alumina.

EXAMPLE 8

3-[5-(3-Isopropyl-1,2,4-oxadiazol)yl]-1,2,5,6-tetrahydropyridine hydrochloride

This was prepared from isopropyl carboxamide oxime and 3-methoxycarbonyl-1-vinyloxy-carbonyl-1,2,5,6-tetrahydropyridine and recrystallised from methanol/diethyl ether, mp 112°C, Rf = 0.28 in dichloromethane/methanol (20:1) on silica.

EXAMPLE 9

3-[3-(1-Hydroxy-1-phenylmethyl)-1,2,4-oxadiazol-5-yl]-quinuclidine. Hydrogen oxalate

a) α- (Tetrahydropyranyloxy)benzyl amide oxime

To a solution of sodium (6.9g, 300mmol) in methanol (1 litre) was added hydroxylamine hydrochloride (20.8g, 300mmol) followed by α-(tetrahydropyranyloxy)benzyl cyanide (43.4g, 200mmol; J.R. Anderson, R.L. Edwards and A.J.S. Whalley, JCS Perkin I, 215, (1982)). After stirring for 16 hours, the reaction was filtered, evaporated, and dissolved in dry ether (1 litre). The following day, a white precipitate formed (35.4g, 71%); mp 90-92°C; $R_f$ = 0.47 in dichloromethane/methanol (9:1) on silica; (Found: C, 62.2; H, 7.2; N, 11.2; $C_{13}H_{18}N_2O_3$ requires C, 62.3; H, 7.2; N, 11.2%); m/e 251 $(M+H)^+$; δ (360MHz, $d_6$-DMSO) 1.46-1.82 (6H, m, 3 x $CH_2$); 3.25-3.32 and 3.40-3.46 (0.33H and 0.67H, each m, one of $CH_2O$); 3.64-3.71 and 3.78-3.84 (0.67H and 0.33H, each m, one of $CH_2O$); 4.49 and 4.71 (0.33H and 0.67H, each t, each J = 3Hz, CHO-); 5.01 and 5.03 (0.67H and 0.33H each s, PhCH); 5.22 and 5.26 (0.67H and 1.33H, each s, $NH_2$); 7.25-7.46 (5H, m, $C_6H_5$); 9.10 and 9.24 (0.33H and 0.67H, each s, (=NOH).

b) 3-[3-(1-Phenyl-1-tetrahydropyranyloxymethyl)-1,2,4-oxadiazol-5-yl]-quinuclidine

The foregoing amide oxime (15.5g, 60mmol) was stirred in dry tetrahydrofuran (200ml) with powdered 4A molecular sieves (5g) under a dry nitrogen atmosphere for 1 hour. Sodium hydride (2.4g of a 60% dispersion in oil, 60mmol) was added and the reaction heated at 50°C for 0.5 hour. A solution of methyl quinuclidine-3-carboxylate (8.0g, 48mmol) in tetrahydrofuran (50ml) was added and the reaction mixture was heated under reflux for 4 hours. The oil obtained after filtration and evaporation of the solvent was purified by column chromatography on silica using dichloromethane/methanol (20:1) to yield the title compound as a pale yellow oil (12.7g, 72%); $R_f$ = 0.34 in dichloromethane/methanol (9:1) on silica; m/e 370 $(M+H)^+$; δ (360MHz, $CDCl_3$) 1.36-1.93 (10H, m, 5 x $CH_2$); 2.21-2.23 (1H, m, 4-CH); 2.83-3.54 (8H, m, 3 x $CH_2N$ and $CH_2O$); 3.75-3.82 and 3.91-3.97 (1H, each m, 3-CH); 4.69-4.71 and 4.85-4.86 (1H, each m, CH); 5.96 and 6.01 (1H, each m, PhCH) and 7.27-7.55 (5H, m, $C_6H_5$).

A small portion of the above product was precipitated as the hydrogen oxalate salt mp 95-100°C; (Found: C, 58.2; H, 6.5; N, 8.7; $C_{21}H_{27}N_3O_3$. $C_2H_2O_4$. $H_2O$ requires C, 57.9; H, 6.5; N, 8.8%).

c) 3-[3-(1-Hydroxy-1-phenylmethyl)-1,2,4-oxadiazol-5-yl]-quinuclidine. Hydrogen oxalate

The foregoing tetrahydropyranylether (11.0g, 29.8mmol) in methanol (250ml) was treated with aqueous hydrochloric acid (2N, 30ml, 60mmol) for 1 hour. The residue after evaporation of the solvents was taken up in dichloromethane and water, cooled and then basified with potassium carbonate. The organic layer was separated and the aqueous was re-extracted several times with dichloromethane. Evaporation of the combined organic extracts gave the title product as its free base (8.1g, 95%); $R_f$ = 0.5 in dichloromethane/methanol (9:1) on alumina, from which the hydrogen oxalate salt was prepared, mp 105-107°C; (Found: C, 54.1; H, 5.6; N, 10.1; $C_{16}H_{19}N_3O_2$. $C_2H_2O_4$. 1.5 $H_2O$ requires, C, 54.0; H, 5.5; N, 10.5%); m/e 286 $(M+H)^+$; δ (360MHz, $D_2O$) 1.6-1.8 (1H, m, one of $CH_2$); 1.85-1.96 (1H, m, one of $CH_2$); 2.06-2.22 (2H, m, $CH_2CH_2$); 2.10-2.14 (1H, m, 4-CH); 3.34-3.43 (4H, m, 2 x $CH_2N$); 3.77-3.79 (2H, m, $CH_2N$); 3.88-3.89 (1H, m, 3-CH); 6.06 (1H, s, PhCH); 7.43-7.49 (5H, m, $C_6H_5$).

EXAMPLE 10

3-[3-(1-Acetoxy-1-phenylmethyl)-1,2,4-oxadiazol-5-yl]-quinuclidine. Hydrogen oxalate

3-[3-(1-Tetrahydropyranyloxy-1-phenylmethyl)-1,2,4-oxadiazol-5-yl]-quinuclidine (369mgs, 1mmol) in acetic acid (10ml) was treated with acetyl chloride (1ml) at room temperature for one hour. Dry toluene (100ml) was added and the solvents removed under vacuum. The residue was basified with cold saturated potassium

carbonate solution and extracted into dichloromethane. The material isolated from the organic extracts (170mgs, 52%) was crystallised as the hydrogen oxalate salt mp 80-82°C; $R_f$ = 0.66 in dichloromethane/methanol (20:1) on alumina; m/e 328 $(M+H)^+$; (Found: C, 57.4; H, 5.6; N, 10.2; $C_{18}H_{21}N_3O_3$. $C_2H_2O_4$ requires C, 57.6; H, 5.6; N, 10.1%); δ (360MHz, $D_2O$ 1.66-1.80 (m, 1H, one of $CH_2$); 1.82-1.92 (1H, m, one of $CH_2$); 2.04-2.22 (2H, m, $CH_2$); 2.22 (3H, s, $CH_3O$); 2.60-2.64 (1H, m, 4-CH); 3.32-3.42 (4H, m, 2 x $CH_2N$); 3.77-3.80 (2H, m, $CH_2N$); 3.89-3.94 (1H, m, 3-CH); 6.95 (1H, s, PhCH) and 7.47-7.54 (5H, m, $C_6H_5$).

## EXAMPLE 11

3-(3-Benzoyl-1,2,4-oxadiazol-5-yl)-quinuclidine. Sesquioxalate

3-[3-(1-Hydroxy-1-phenylmethyl)-1,2,4-oxadiazol-5-yl]-quinuclidine (8.1g, 28.4mmol) in dichloromethane (500ml) was stirred with activated manganese dioxide (50g). After 0.5 hour, the reaction mixture was filtered and the manganese dioxide repeatedly washed with dichloromethane. The solution obtained was again filtered through Florisil (100-120 mesh) and evaporated to yield the title compound as its free base (6.1g, 75%); $R_f$ = 0.18 in dichloromethane/methanol (99:1) on alumina. This material in ether was crystallised as the sesquioxalate salt, mp 82-85°C; (Found: C, 53.1; H, 4.8; N, 10.2; $C_{16}H_{17}N_3O_2$. 1.5 $C_2H_2O_4$. 0.5 $H_2O$ requires C, 53.4; H, 4.9; N, 9.8%); δ (360MHz, $D_2O$) 1.90-1.97 (2H, m, $CH_2$); 2.16-2.23 (2H, m, $CH_2$); 2.73-2.76 (1H, m, 4-CH); 3.40-3.52 (4H, m, 2 x $CH_2N$); 3.79-3.92 (2H, m, $CH_2N$); 4.10-4.11 (1H, m, 3-CH); 7.6 (2H, t, J = 8Hz, 3-H and 5-H of $C_6H_5$); 7.84 (1H, t, J = 7Hz, 4-H of $C_6H_5$) and 8.20-8.22 (2H, dd, J = 7 and 1Hz, 2-H and 6-H of $C_6H_5$).

## EXAMPLE 12

3-[3-(1-Hydroxy-1-phenylethyl)-1,2,4-oxadiazol-5-yl]-quinuclidine. Hydrogen oxalate

3-(3-Benzoyl-1,2,4-oxadiazol-5-yl)-quinuclidine (1.0g, 3.5mmol) in dry THF under an atmosphere or dry nitrogen was treated with methyl magnesium chloride at room temperature for 0.5 hour before quenching with saturated ammonium chloride solution. The organic layer was separated and the aqueous extracted several times with dichloromethane. The combined extracts were filtered through Florisil and evaporated to give a white solid (1.0g, 97%), mp 114-117°C; $R_f$ = 0.1 in dichloromethane/methanol (9:1) on silica. The solid in ether was treated with etherial oxalic solution to afford the hydrogen oxalate salt mp 55-60°C; (Found: C, 56.5; H, 6.1; N, 10.0 $C_{17}H_{21}N_3O_2$. $C_2H_2O_4$. 0.9 $H_2O$ requires C, 56.3; H, 6.2; N, 10.4%); δ (360MHz, $D_2O$) 1.73-2.14 (4H, 2 x $CH_2$); 1.99 (3H, s, $CH_3$); 2.61-2.62 (1H, m, 4-CH); 3.35-3.42 (4H, m, 2 x $CH_2N$); 3.75-3.79 (2H, m, $CH_2N$); 3.84-3.88 (1H, m, 3-CH) and 7.37-7.50 (5H, m, $C_6H_5$).

## EXAMPLE 13

3-[3-(1,1-Diphenyl-1-hydroxymethyl)-1,2,4-oxadiazol-5-yl]-quinuclidine. Hydrogen oxalate.

3-(3-Benzoyl-1,2,4-oxadiazol-5-yl)-quinuclidine (566 mgs, 2 mmol ) in dry THF under an atmosphere of dry nitrogen was treated with phenyl magnesium bromide (2 ml of a 3M solution, 6 mmol) at room temperature for 0.5 hours. Saturated ammonium chloride was added and the mixture partitioned and extracted twice with dichloromethane. The material isolated from the combined organic extracts was purified by column chromatography using neutral alumina with dichloromethane/methanol (99:1) to yield the free base of the title compound as a pale yellow oil (361mgs, 58%); $R_f$ = 0.86 in dichloromethane/methanol (9:1) on alumina. The foregoing oil was crystallised as the hydrogen oxalate salt, m.p. 65-70°C; (Found: C, 61.0; H, 5.4; N, 8.8; $C_{22}H_{23}N_3O_2$. $C_2H_2O_4$. 1.1 $H_2O$ requires C, 61.2; H, 5.8; N, 8.9%); m/e 362 $(M + H)^+$; δ(360 MHz, $D_2O$). 1.72 - 2.24 (4H, m, 2 x $CH_2$); 2.63 - 2.64 (1H, m, 4-CH); 3.35 - 3.42 and 3.77 -3.82 (6H, m, 3 x $CH_2N$); 3.92 - 3.94 (1H, m, 3-CH) and 7.36 - 7.44 (10H, m, 2 x $C_6H_5$)

## EXAMPLE 14

3-[3-(2-Methoxy-1-phenylethenyl)-1,2,4-oxadiazol-5-yl]-quinuclidine. Hydrogen chloride.

Methoxymethyl triphenyl phosphonium chloride (2.9 g, 8.5 mmol) was treated with phenyl lithium (1.7M, 5 ml, 8.5 mmol) in tetrahydrofuran (200 ml) at room temperature. After 1 hour, the reaction mixture was cooled to -60°C and 3-(3-benzoyl-1,2,4-oxadiazol-5-yl)-quinuclidine (1.6 g, 5.6 mmol) in tetrahydrofuran (50 ml) was added dropwise. The reaction was maintained at -60°C for 0.5 hours, and then stirred at room temperature for 4 hours. The residue after filtration and evaporation was triturated with ether to precipitate triphenyl

phosphine oxide and the supernatant treated with excess ethereal hydrogen chloride. This was recrystallised from propane-2-ol/diethyl ether to give the title produce as a white solid (900 mgs, 35%); m.p. = 199 - 200°C; (Found: C, 61.7; H, 6.4; N, 12.0; $C_{18}H_{21}O_2$. HCl. 0.1. $H_2O$ requires C, 61.8; H, 6.4; N, 12.0%.); m/e 312 (M + H)$^+$; δ (360 MHz, $D_2O$) 1.92 - 2.18 (4H, m, 2 x $CH_2$); 2.65 - 2.67 (1H, m, 4-CH); 3.38 - 3.46 (4H, m, 2 x $CH_2N$); 3.81 - 3.96 (3H, m, 1 x $CH_2N$ and 3-CH); 3.89 and 3.96 (3H, each s, $OCH_3$); 7.08 and 7.56 (1H, each s, CH = C) and 7.35 - 7.50 (5H, m, $C_6H_5$).

## EXAMPLE 15

3-[5-(1-Hydroxy-1-phenylmethyl)-1,2,4-oxadiazol-3-yl]-quinuclidine. Hydrogen oxalate

a) Quinuclidine-3-carboxamide oxime

Sodium metal (1.46 g, 53 mmol) was dissolved in dry methanol (500 ml) and hydroxylamine hydrochloride (4.4 g, 64 mmol) added followed by quinuclidine-3-carbonitrile (7.2 g, 53 mmol). After stirring for 16 hours, the reaction mixture was filtered and evaporated. The amide product was purified by chromatography on alumina to yield the title compound as a pale yellow solid (5.2 g, 58%); m.p. 160-162°C; $R_f$ = 0.15 in dichloromethane/methanol (9:1) on silica: (Found: C, 56.0; H, 8.7; N, 24.0; $C_8H_{15}N_3O$.0.15 $H_2O$ requires C, 55.9; H, 8.8; N, 24.4 %); m/e 170 (M + H)$^+$; δ (360 MHz, $d_6$-DMSO) 1.19 - 1.95 (4H, m, 2 x $CH_2$); 2.26 -2.32 (1H, m, 4-CH); 2.49 - 2.73 (6H, m, 3 x $CH_2N$); 3.11 (1H, ddd, J = 2, 7 and 8 Hz, 3-CH); 3.35 (2H, broad s, (exch. $D_2O$), $NH_2$); 9.0 (1H, broad s, (exch. $D_2O$), NOH).

b) 3-[5-(1-Phenyl-1-tetrahydropyranyloxymethyl)-1,2,4-oxadiazol-3-yl]-quinuclidine

The foregoing amide oxime 95 g, 29.6 mmol) was stirred in dry THF (200 ml) with powdered molecular sieves (5 g) under a dry nitrogen atmosphere for 0.5 hours. Sodium hydride (1.18 g of a 60% dispersion in oil, 29.6 mmol) was added, the solution heated to reflux for 15 min and then α-tetrahydropyranyloxy-phenylacetic acid methyl ester (7.25 g, 29.6 mmol) in dry THF (100 ml) was added and the reaction heated to reflux for 2 hours. The reaction was filtered and evaporated and the residue chromatographed on silica using dichloromethane/methanol (9:1) to yield the free base of the title oxadiazole, (3.5 g, 36%) $R_f$ = 0.35 in dichloromethane/methanol on silica; m/e 370 (M + H)$^+$; δ(360 MHz, $CDCl_3$) 1.32 - 1.93 (10H, m, 5 x $CH_2$); 2.26 - 2.3 (1H, m, 4-CH); 2.96 - 3.24 (8H, m, 3 x $CH_2H$ and 1 x $CH_2O$); 3.69 - 3.75 and 3.80 -3.90 (1H, each m, 3-CH); 4.69 - 4.70 and 4.85 - 4.87 (1H, each m, CH (OCH)$OCH_2$); 5.99 and 6.01 (1H, each s, PhCH) and 7.22 - 7.56 (5H, m, $C_6H_5$). For analysis a small portion of the above product was precipitated as the sesqui oxalate salt m.p. 78-89°C; (Found: C, 56.8; H, 6.3; N, 8.0; $C_{21}H_{27}N_3O_3$. 1.5 ($C_2H_2O_4$) requires C, 57.1; H, 6.0; N, 8.3%).

c) 3-[5-(1-Hydroxy-1-phenylmethyl)-1,2,4-oxadiazol-3-yl]-quinuclidine. Hydrogen oxalate.

The foregoing tetrahydropyranyl ether (3.2 g, 8.7 mmol) in methanol (100 ml) was treated with aqueous hydrochloric acid (2N, 10 ml, 20 mmol). After stirring for 1.5 h, the solvent was evaporated off and the residue basified with saturated potassium carbonate and extracted with dichloromethane. Evaporation of the organic extracts gave the title product, (2.2 g, 89%); $R_f$ = 0.53 in dichloromethane/methanol (9:1) on alumina. This material in ether was treated with an excess of etherial oxalic acid and solution and recrystallised from i-propanol to afford the hydrogen oxalate salt, m.p. = 75 °C (dec); (Found: C, 55.7; H, 5.8; N, 10.4; $C_{16}H_{19}N_3O_2$. $C_2H_2O_4$. 0.75 $H_2O$ requires C, 55.6; H, 5.8; N, 10.8%; m/e 286 (M + H)$^+$; δ (360 MHz, $D_2O$); 1.8 - 2.2 (4H, m, 2 x $CH_2$); 2.50 - 2.53 (1H, m, 4-CH); 3.28 - 3.45 (4H, m, 2 x $CH_2N$); 3.62 - 3.78 (3H, m, 3-CH and $CH_2N$); 6.18 (1H, s, PhCH) and 7.45 - 7.52 (5H, m, $C_6H_5$).

## EXAMPLE 16

3-[3-(1,1-Diphenyl-1-hydroxymethyl)-1,2,4-oxadiazol-5-yl]-1,2,5,6-tetrahydropyridine. Hydrogen oxalate

a) 1-t-Butyloxycarbonyl-3-methoxycarbonyl-1,2,5,6-tetrahydropyridine

1-Vinyloxycarbonyl-3-methoxycarbonyl-1,2,5,6-tetrahydropyridine (69.70g, 0.33mol; R.A. Olofson et al, Tet. Lett., 1563-66, (1977)) was left standing in saturated methanolic hydrogen chloride (600ml) for 16 hours. The reaction mixture was evaporated in vacuo and the residue dried over phosphorus pentoxide for 16 hours. The solid obtained was suspended in dry dichloromethane (1 litre), triethylamine (92ml, 0.66mol) added and the mixture stirred for 15 minutes. Di-t-butyldicarbonate (96.0g, 0.44mol) was added over 15 minutes, then the reaction was stirred for 6 hours at room temperature, filtered, washed with 0.5M HCl (2 x 500ml), water (2 x 500ml) and finally saturated sodium bicarbonate (1 x 500ml). The resulting organic layer was dried (sodium sulphate) and then evaporated to give an orange oil which was distilled under reduced pressure to yield the required tetrahydropyridine as a pale yellow oil (65.2g, 82%); bp 134-136°C (1.5mmHg); $R_f$ = 0.75 in ethyl acetate on silica; (Found: C, 59.5; H, 7.9; N, 5.7; $C_{12}H_{19}NO_4$ requires C, 59.7; H, 7.9; N, 5.8%); m/e 240 (M-H)$^+$;

δ (360MHz, $CDCl_3$) 1.48 (9H, s, $C_4H_9$); 2.26-2.36 (2H, m, $=CHCH_2$); 3.48 (2H, dd, J = 6Hz, $CH_2N$); 3.76 (3H, s, $OCH_3$); 4.08-4.14 (2H, m, $CH_2N$); 7.02-7.10 (1H, m, =CH).

b) 1-t-Butyloxycarbonyl-3-[3-(1-phenyl-1-tetrahydropyranyloxymethyl)-1,2,4-oxadiazol-5-yl]-1,2,5,6-tetrahydropyridine

α-(Tetrahydropyranyloxy)benzyl amide oxime (7.5g, 30mmol) was stirred in dry tetrahydrofuran (100ml) with powdered 4A molecular sieves (5g) under a dry nitrogen atmosphere for 1 hour. Sodium hydride (1.2g of 60% dispersion in oil, 30mmol) was added and the reaction heated to reflux for 0.5 hour. The reaction was cooled and a solution of the foregoing ester (7.2g, 30mmol) in dry tetrahydrofuran (50ml) was added and the reaction mixture heated to reflux for 2 hours. The oil obtained after filtration and evaporation of the solvent was purified by column chromatography on silica using dichloromethane/methanol (99:1) to yield an oil (4.3g, 32%); $R_f$ = 0.49 in dichloromethane/methanol on silica; this was used directly in the next step.

c) 1-t-Butyloxycarbonyl-3-[3-(1-hydroxy-1-phenylmethyl)-1,2,4-oxadiazol-5-yl]-1,2,5,6-tetrahydropyridine

The foregoing oxadiazole (4g, 9.1mmol) in ethanol (100ml) and water (0.5ml) was treated with pyridine p-toluene sulphonate (500mg, 2mmol) and the reaction mixture stirred at 55° C for 16 hours. The residue after evaporation of solvent was partitioned between ethyl acetate and water. Evaporation of the combined organic extracts and purification by column chromatography on silica using dichloromethane/methanol (98:2) gave an oil (2.0g, 62%); $R_f$ = 0.15 in dichloromethane/methanol (99:1) on silica; m/e 358 $(M+H)^+$; δ (360MHz, $CDCl_3$) 1.23-2.0 (10H, m, 5 x $CH_2$); 1.48 (9H, s, $C_4H_9$); 2.32-2.41 (2H, m, $CH_2C=$); 3.52-3.57 and 3.76-4.02 (4H, each m, $CH_2O$ and $CH_2N$); 4.32 (2H, broad s, $=CCH_2N$); 4.71 and 4.84 (1H, each t, each J = 3Hz, OCHO); 5.97 and 6.03 (1H, each s, PhCH); 7.16 (1H, broad s, CH=) and 7.26-7.56 (5H, m, $C_6H_5$).

d) 1-t-Butyloxycarbonyl-3-(3-benzoyl-1,2,4-oxadiazol-5-yl)-1,2,5,6-tetrahydropyridine

The foregoing product (1.9g, 5.3mmol) in dichloromethane (100ml) was treated at 20° with maganese dioxide (10g) for 0.5 hour. Filtration and evaporation of the organic solvent yielded an oil (1.9g, 100%); $R_f$ = 0.46 in dichloromethane/methanol (99:1) on silica.

e) 1-t-Butyloxycarbonyl-3-[3-(1,1-diphenylhydroxymethyl)-1,2,4-oxadiazol-5-yl]-1,2,5,6-tetrahydropyridine

The foregoing product (1.8g, 5.1mmol) in dry tetrahydrofuran (50ml) under an atmosphere of dry nitrogen was treated with phenyl magnesium bromide (3ml of a 3M solution in ether, 9mmol) for 1 hour before quenching with saturated ammonium chloride. The organic layer was separated and the aqueous extracted several times with dichloromethane. The combined extracts were dried ($MgSO_4$), filtered and evaporated and the residue purified by column chromatography to give the title compound as an oil (1.0g, 48%); $R_f$ = 0.22 in dichloromethane/methanol (99:1) on silica; (Found: C, 67.3; H, 6.2; N, 9.1; $C_{25}H_{27}N_3O_4$. $0.6H_2O$ requires C, 67.6; H, 6.4; N, 9.5%); m/e 416 $(M-OH)^+$; δ (360MHz, $CDCl_3$) 1.48 (9H, s, $C_4H_9$); 2.36-2.44 (2H, m, $CH_2CH=$); 3.56 (2H, dd, J = 6Hz, $CH_2N$); 4.37 (2H, broad s, $NCH_2C=$) and 7.3-7.6 (11H, m, 2 x $C_6H_5$ and CH=).

f) 3-[3-(1,1-Diphenyl-1-hydroxymethyl)-1,2,4-oxadiazol-5-yl]-1,2,5,6-tetrahydropyridine. Hydrogen oxalate

The foregoing N-protected tetrahydropyridine (0.9g, 2.1mmol) was stirred in dichloromethane (50ml) and treated with trifluoroacetic acid (2.85g, 25mmol) for 16 hours. The solvents were removed and the residue partitioned between cold saturated potassium carbonate and dichloromethane. The material isolated from the organic layer (560mgs, 80%; $R_f$ = 0.18 in dichloromethane/methanol (99:1) on alumina) was treated with etherial oxalic solution to afford the hydrogen oxalate salt mp 118-120° C (dec); (Found C, 58.9; H, 5.1; N, 8.70; $C_{20}H_{19}N_3O_2$. 1.5 $(C_2H_2O_4)$ requires C, 59.0; H, 4.7; N, 9.0%); m/e 334 $(M+H)^+$; δ (360MHz, $D_2O$) 2.6-2.8 (2H, m, $=CHCH_2$); 3.42-3.45 (2H, m, $NCH_2$); 4.1-4.16 (2H, m, $NCH_2C=$) and 7.32-7.44 (11H, m, 2 x $C_6H_5$ and =CH).

## EXAMPLE 17

3-[3-(1-Hydroxy-1-phenylmethyl)-1,2,4-oxadiazol-5-yl]-1,2,5,6-tetrahydropyridine. Trifluoroacetate

The 1-t-butyloxycarbonyl-1,2,5,6-tetrahydro pyridine derivative prepared in Example 16c (250mgs) in dichloromethane (2ml) was treated at 20° for 16 hours with trifluoroacetic acid (1ml). Evaporation of the solvents followed by exhaustive trituration with anhydrous ether to give a gum which solidified as a white foam on drying; mp 45-47°; δ (250MHz, $d_6$-DMSO) 2.42-2.55 (2H, m, $CH_2$); 3.08-3.22 (2H, m, $CH_2CH_2N$); 3.82-3.98 (2H, m, $NCH_2C=$); 5.76-5.84 (1H, s, PhCH); 6.24-6.56 (1H, broad s, OH): 7.06-7.15 (1H, m, CH=) and 7.16-7.40 (5H, m, $C_6H_5$).

## EXAMPLE 18

3-[5-(3-[4-Chlorophenyl]-1,2,4-oxadiazol)-yl]-1,2,5,6-tetrahydropyridine hydrochloride

Sodium hydride (0.68g of a 55% dispersion in oil, 16mmol) was added to a stirred suspension of 4-chlorophenylcarboxamide oxime (3.62g, 21mmol) and 4A molecular sieves (30g) in anhydrous tetrahydrofuran (60ml) under a nitrogen atmosphere, and the reaction mixture heated at 50°C for 0.75 hours. 3-Methoxycarbonyl-1-vinyloxycarbonyl-1,2,5,6-tetrahydropyridine (R.A. Olofson et al, Tet. Letts. 1977, 1567, 3.0g, 14mmol) was added and the reaction mixture was stirred whilst heating under reflux for 2 hours. The reaction mixture was cooled, water added and the organic layer collected. The aqueous extract was extracted with dichloromethane (5 times) and the combined organics were dried (sodium sulphate) and evaporated to give an orange solid. The product was purified by colum chromatography on neutral alumina using dichloromethane/methanol (gradient, 100:1 to 10:1). A second column on alumina using ethyl acetate as eluent gave the title compound free base. The hydrochloride salt had mp 292°C (methanol/diethyl ether); (Found: C, 51.92; H, 4.46; N, 13.76. $C_{13}H_{12}ClN_3O.HCl$ requires C, 52.36; H, 4.39; N, 14.09%); m/e 260 $(M-H)^+$ of free base); δ ($D_2O$, 360MHz) 2.76-2.85 (2H, m, 5-$CH_2$), 3.48 (2H, dd, J = 6Hz, 6-$CH_2$), 4.18-4.28 (2H, m, 2-$CH_2$), 7.40-7.46 (1H, m, 4-CH), 7.56-7.66 and 7.94-8.02 (each 2H, each m, 4 x phenyl H).

## EXAMPLE 19

exo-3-[5-(3-Isopropyl-1,2,4-oxadiazol)-yl]-endo-5-hydroxy-1-azabicyclo[2.2.1]heptane hydrogen oxalate

a) trans-3,4-Dimethoxycarbonylpyrrolidine

This was prepared from glycine and dimethylfumarate by the procedure reported by Joucla et al, J. Chem. Soc. Chem. Commun., (1985), 1566.

b) 1-Methoxycarbonylmethyl-trans-3,4-dimethoxycarbonyl pyrrolidine

A solution of trans-3,4-dimethoxycarbonyl pyrrolidine (4.1g, 22mmol) in xylene (30ml) was added to a rapidly stirred suspension of potassium carbonate (7g) in xylene (150ml), at 120°C. After 0.25 hour, a solution of methylbromoacetate (3.45g, 22.5mmol) in xylene (30ml) was added dropwise and the mixture stirred rapidly at 140°C for 2 hours. The solution was decanted from the inorganic residue which was taken up into water (100ml) and extracted with dichloromethane (3 x 150ml). The combined organics were dried (sodium sulphate) and the solvent removed under vacuum to give the title triester as a yellow liquid (6g); δ (360MHz, $CDCl_3$) 2.96-3.11 (4H, m, $2CH_2$ and $5CH_2$), 3.31 (1H, d, J = 16.5Hz, one of $NCH_2$), 3.38 (1H, d, J = 16.5Hz, one of $NCH_2$), 3.46-3.52 (2H, m, 3CH and 4CH), 3.74 (9H, s, 3 x $CH_3$).

c) 3-Methoxycarbonyl-5,5-dimethoxy-1-azabicyclo[2.2.1]heptane

A solution of 1-methoxycarbonylmethyl-trans-3,4-dimethoxycarbonylpyrrolidine (5g, 19.31mmol) in toluene (75ml) was added dropwise over a 1 hour period to a rapidly stirred solution of potassium-t-butoxide (9g, 80mmol) in toluene (250ml) at 130°C. The mixture was refluxed for 4 hours, cooled to room temperature and concentrated hydrochloric acid (75ml) added dropwise and stirred for 0.25 hours. The organic phase was extracted with further portions of hydrochloric acid (3 x 50ml) and the combined aqueous heated at 110°C for 16 hours. The solvent was then removed in vacuo, the residue dried and taken up into methanol (saturated with hydrogen chloride, 150ml). The mixture was stirred at room temperature for 24 hours and the solvent removed under vacuum. The residue was dissolved in water (50ml), basified to pH > 10 with potassium carbonate and extracted with dichloromethane (5 x 150ml). The combined extracts were dried (sodium sulphate) and the residue remaining after removal of the solvents was chromatographed through silica-gel, using dichloromethane/methanol (93:7) as eluant, to give the title ester as a yellow liquid (0.5g). An analytical sample was prepared as the hydrogen oxalate salt, mp 134.5-136.5°C (propan-2-ol); (Found: C, 47.04; H, 6.20; N, 4.50. $C_{10}H_{17}NO_4.(CO_2H)_2$ requires C, 47.21; H, 6.27; N, 4.59%); δ (360MHz, $CDCl_3$) 2.44 (1H, dd, J = 9.8 and 3.2Hz), 2.63 (1H, dd, J = 12.7 and 3.2Hz), 2.77 (1H, d, J = 12.7Hz), 2.80-3.10 (5H, m), 3.11 (3H, s, $OCH_3$), 3.24 (3H, s, $OCH_3$), 3.71 (3H, s, $CO_2CH_3$).

d) exo-3-[5-(3-Isopropyl-1,2,4-oxadiazol)-yl]-5,5-dimethoxy-1-azabicyclo[2.2.1]heptane

Sodium hydride (0.34g of a 55% dispersion in oil, 7.7mmol) was added to a stirred suspension of isopropylcarboxamide oxime (0.714g, 7.0mmol) and 4A molecular sieves (10g) in anhydrous tetrahydrofuran (50ml) under a nitrogen atmosphere. The reaction mixture was stirred at 55°C for 1.5 hours. The preceding ester (1.0g, 4.65mmol) was added and the reaction mixture was stirred whilst heating under reflux for 3 hours, then cooled to room temperature. Water (30ml) was added followed by dichloromethane (50ml) and the mixture was stirred for 10 minutes then filtered. The organic layer was separated and the aqueous re-extracted with dichloromethane. The combined organics were dried (sodium sulphate) then evaporated to dryness. The residue was dissolved in a minimum quantity of methanol and sodium methoxide (0.27g, 5mmol) was added. After stirring for 2 hours the methanol was evaporated and the residue taken up into dichloromethane, washed with water, dried (sodium sulphate) and evaporated to dryness to give an orange oil which was purified by column chromatography on silica using dichloromethane/methanol (50:1). The title compound was obtained as a pale yellow oil (0.34g, 27%); m/e 252 $(M-CH_3)^+$; $CI^-$, m/e 266 $(M-H)^-$. (Found: $CI^-$, $(M-H)^-$, 266.1524.

$C_{13}H_{20}N_3O_3$ requires 266.1505); δ ($CDCl_3$, 250MHz) 1.33 (6H, d, J = 7Hz, 2 x $CH_3$), 2.45 (dd, J = 2,15Hz), 2.77 (dd, J = 2,10Hz) and 2.90-3.20 (total 8H, m, isopropyl-CH, 2-$CH_2$, 4-CH, 6-$CH_2$ and 7-$CH_2$), 3.23 (3H, s, $OCH_3$), 3.27 (3H, s, $OCH_3$), 3.46-3.56 (1H, m, 3-CH).

e) exo-3-[5-(3-Isopropyl-1,2,4-oxadiazol)-yl]-1-azabicyclo[2.2.1]heptan-5-one

Ice cold perchloric acid (70% solution in water, 8ml) was added to the preceding ketal (0.33g, 1.24mmol) with stirring and heated at 85°C for 0.5 hour. The solution was cooled, water (40ml) and dichloromethane (60ml) were added and the aqueous basified to pH 10 with potassium carbonate. The mixture was filtered through hyflo and the organic layer separated. The aqueous was re-extracted with dichloromethane (twice) and the combined organics were dried (sodium sulphate) then evaporated to give a yellow oil which crystallised on standing (0.26g, 95%), mp 51-52°C. Rf = 0.24 in dichloromethane/methanol (9:1) on silica; m/e 222 $(M+H)^+$; (Found: $(M+H)^+$, 222.1229. $C_{11}H_{16}N_3O_2$ requires 222.1242); $\nu_{max}$ (nujol) 1750$cm^{-1}$; δ ($CDCl_3$, 360MHz) 1.34 (6H, d, J = 7Hz, 2 x $CH_3$), 2.93 (dd, J = 4,18Hz) and 3.02-3.32 (total 8H, m, isopropyl-CH, 2-$CH_2$, 4-CH, 6-$CH_2$ and 7-$CH_2$), 3.40-3.55 (1H, m, 3-CH).

f) exo-3-[5-(3-Isopropyl-1,2,4-oxadiazol)-yl]-endo-5-hydroxy-1-azabicyclo[2.2.1]heptane hydrogen oxalate

Sodium borohydride (79mg, 2mmol) was added to an ice cooled solution of the preceding ketone (230mg, 1mmol) in ethanol (30ml) and stirred for 0.5 hour then allowed to warm to room temperature and stirred for a further 0.5 hour. The solvent was evaporated and the residue taken up into dichloromethane. Water was added and basified to pH 10 with potassium carbonate. The organic layer was separated and the aqueous re-extracted with dichloromethane (three times). The combined organics were dried (sodium sulphate) and evaporated to afford a yellow oil (200mg, 90%). The hydrogen oxalate salt had mp 174-178°C (methanol). (Found: C, 49.75; H, 6.11; N, 13.36. $C_{11}H_{17}N_3O_2$. $C_2H_2O_4$ requires C, 49.84; H, 6.11; N, 13.41%); m/e 223 ($M^+$ of free base); (Found: $M^+$ (free base), 223.1308. $C_{11}H_{17}N_3O_2$ requires 223.1321); δ ($D_2O$, 360MHz) 1.29 (6H, d, J = 7Hz, 2 x $CH_3$), 2.96 (1H, dt, J = 3,12Hz, 6-CH), 3.12 (1H, septet, J = 7Hz, isopropyl-CH), 3.40 (1H, d, J = 4.5Hz, 4-CH), 3.45 (1H, d, J = 10Hz, 7-CH), 3.51 (1H, dd, J = 2.5, 10Hz, 7-CH), 3.76-3.98 (3H, m, 2-$CH_2$ and 6-CH), 4.35 (1H, dd, J = 6,8Hz, 3-CH), 4.77-4.86 (1H, m, 5-CH).

## EXAMPLE 20

exo-3-[5-(3-Cyclopropyl-1,2,4-oxadiazol)-yl]-endo-5-hydroxy-1-azabicyclo[2.2.1]heptane hydrogen oxalate

a) exo-3-[5-(3-Cyclopropyl-1,2,4-oxadiazol)-yl]-5,5-dimethoxy-1-azabicyclo[2.2.1]heptane

The title compound was obtained (0.76g, 62%) from 3-methoxycarbonyl-5,5-dimethoxy-1-azabicyclo[2.2.1] heptane (1.0g, 4.65mmol) and cyclopropylcarboxamide oxime (0.70g, 7mmol) as described in Example 19d. Rf = 0.55 in dichloromethane/methanol (8:1) on silica; m/e 266 $(M+H)^+$; (Found: $(M+H)^+$, 266.1530. $C_{13}H_{20}N_3O_3$ requires 266.1505); δ ($CDCl_3$ 360MHz) 1.00-1.07 (4H, m, 2 x cyclopropyl-$CH_2$), 2.01-2.12 (1H, m, cyclopropyl-CH), 2.42 (dd, J = 3.13Hz), 2.72 (dd, J = 3,10Hz), 2.89-3.02 (m) and 3.03-3.18 (total 7H, m, 2-$CH_2$, 4-CH, 6-CH, 6-$CH_2$ and 7-$CH_2$), 3.21 (3H, s, $OCH_3$), 3.26 (3H, s, $OCH_3$), 3.40-3.45 (1H, m, 3-CH).

b) exo-3-[5-(3-Cyclopropyl-1,2,4-oxadiazol)-yl]-1-azabicyclo[2.2.1]heptan-5-one

The title compound was obtained (115mg, 22%) from perchloric acid (70% solution, 15ml) and the foregoing ketal (0.64g, 2.4mmol) as described in Example 19e, except that the reaction mixture was heated for 1.5 hours and the product purified by column chromatography on silica using dichloromethane/methanol (50:1). Rf = 0.25 in dichloromethane/ methanol (10:1) on silica; $\nu_{max}$ (film) 1760$cm^{-1}$; m/e, $CI^+$, 220 $(M+H)^+$; (Found: $(M+H)^+$, 220.1078. $C_{11}H_{14}N_3O_2$ requires 220.1086): δ ($CDCl_3$, 250MHz) 0.94-1.14 (4H, m, 2 x cyclopropyl-$CH_2$), 1.92-2.18 (1H, m, cyclopropyl-CH), 2.90 (dd, J = 4,17Hz) and 3.02-3.64 (total 8H, m, 2-$CH_2$, 3-CH, 4-CH, 6-$CH_2$ and 7-$CH_2$).

c) exo-3-[5-(3-Cyclopropyl-1,2,4-oxadiazol)-yl]-endo-5-hydroxy-1-azabicyclo[2.2.1]heptane hydrogen oxalate

The title compound free base was obtained (50mg, 45%) from the preceding ketone (110mg, 0.5mmol) and sodium borohydride (38mg, 1mmol) as described in Example 19f. The hydrogen oxalate salt had mp 190-192°C (methanol). (Found: C, 49.91; H, 5.56; N, 13.31. $C_{11}H_{15}N_3O_2.C_2H_2O_4$ requires C, 50.16; H, 5.50; N, 13.50%); m/e 221 ($M^+$ of free base); (Found: $M^+$ (free base), 221.1149. $C_{11}H_{15}N_3O_2$ requires 221.1164): δ ($D_2O$, 360MHz) 0.95-1.00 (2H, m, cyclopropyl-$CH_2$), 1.10-1.17 (2H, m, cyclopropyl-$CH_2$), 2.07-2.15 (1H, m, cyclopropyl-CH), 2.94 (1H, dt, J = 3.5, 12.5Hz, 6-CH), 3.37 (1H, d, J = 5Hz, 4-CH), 3.43 (1H, d, J = 10Hz, 7-CH), 3.50 (1H, dd, J = 3,10Hz, 7-CH), 3.76-3.94 (3H, m, 2-$CH_2$ and 6-CH), 4.31 (1H, dd, J = 5.5, 8.5Hz, 3-CH), 4.76-4.85 (1H, m, 5-CH).

## EXAMPLE 21

exo-3-[5-(3-n-Propyl-1,2,4-oxadiazol)-yl]-endo-5-hydroxy-1-azabicyclo[2.2.1]heptane hydrogen oxalate

a) exo-3-[5-(3-n-Propyl-1,2,4-oxadiazol)-yl]-5,5-dimethoxy-1-azabicyclo[2.2.1]heptane

The title compound was obtained (0.14g, 20%) from 3-methoxycarbonyl-5,5-dimethoxy-1-azabicyclo[2.2.1]heptane (0.58g, 2.7mmol) and butanamide oxime (0.413g, 4.0mmol) as described in Example 19d. Rf = 0.48 in dichloromethane/methanol (8:1) on silica; m/e 268 $(M+H)^+$; δ ($CDCl_3$, 250MHz) 0.99 (3H, t, J = 7.5Hz, $CH_3$), 1.75 (2H, sextet, J = 7.5Hz, $CH_2CH_2CH_3$), 2.68 (2H, t, J = 7.5Hz, $CH_2CH_2CH_3$), 2.44 (dd, J = 4, 12.5Hz), 2.75 (dd, J = 4,10Hz) and 2.92-3.20 (total 7H, 2-$CH_2$, 4-CH, 6-$CH_2$ and 7-$CH_2$), 3.22 (3H, s, $OCH_3$), 3.27 (3H, s, $OCH_3$), 3.46-3.54 (1H, m, 3-CH).

b) exo-3-[5-(3-n-Propyl-1,2,4-oxadiazol)-yl]-1-azabicyclo[2.2.1]heptan-5-one

The title compound was obtained (55mg, 56%) from perchloric acid (70% solution, 3ml) and the foregoing ketal (120mg, 0.45mmol) as described in Example 19e, m/e 222 $(M+H)^+$; (Found: $(M+H)^+$, 222.1260. $C_{11}H_{16}N_3O_2$ requires 222.1243): δ ($CDCl_3$, 250MHz) 1.00 (3H, t, J = 7.5Hz, $CH_3$), 1.77 (2H, sextet, J = 7.5Hz, $CH_2CH_2CH_3$), 2.69 (2H, t, J = 7.5Hz, $CH_2CH_2CH_3$), 2.91 (dd, J = 4.5, 18Hz) and 3.08-3.54 (total 8H, m, 2-$CH_2$, 3-CH, 4-CH, 6-$CH_2$ and 7-$CH_2$).

c) exo-3-[5-(3-n-Propyl-1,2,4-oxadiazol)-yl]-endo-5-hydroxy-1-azabicyclo[2.2.1]heptane hydrogen oxalate

The title compound free base was obtained (36mg, 70%) from the preceding ketone (52mg, 0.23mmol) and sodium borohydride (18mg, 0.47mmol) as described in Example 19f. The hydrogen oxalate salt had mp 165-168°C (methanol). (Found: C, 49.84; H, 6.15; N, 13.33. $C_{11}H_{17}N_3O_2.C_2H_2O_4$ requires C, 49.84; H, 6.11; N, 13.41%); m/e 224 $(M+H)^+$ of free base; (Found: $(M+H)^+$ free base, 224.1407. $C_{11}H_{18}N_3O_2$ requires 224.1399); δ ($D_2O$, 250MHz) 0.93 (3H, t, J = 7Hz, $CH_3$), 1.73 (2H, sextet, J = 7Hz, $CH_2CH_2CH_3$), 2.74 (2H, t, J = 7Hz, $CH_2CH_2CH_3$), 2.97 (1H, dt, J = 3,9Hz, 6-CH), 3.40 (1H, d, J = 4.5Hz, 4-CH), 3.45 (1H, d, J = 10.5Hz, 7-CH), 3.50 (1H, dd, J = 3,10.5Hz, 7-CH), 3.76-3.95 (3H, m, 2-$CH_2$ and 6-CH), 4.35 (1H, dd, J = 7,8.5Hz, 3-CH), 4.79-4.85 (1H, m, 5-CH).

## EXAMPLE 22

exo-3-[5-(3-n-Propyl-1,2,4-oxadiazol)-yl]-1-azabicyclo[2.2.1]heptane hydrochloride

Prepared from 3-methoxycarbonyl-1-azabicyclo[2.2.1]heptane and butanamide oxime as described in Example 1. The hydrochloride salt had mp 119-120.3°C (propan-2-ol/diethyl ether); Rf = 0.35 in 10% methanol/dichloromethane on silica. (Found: C, 52.75; H, 7.51; N, 16.68. $C_{11}H_{17}N_3O$. HCl.0.4$H_2O$ requires C, 52.65; H, 7.55; N, 16.75%); m/e 207 ($M^+$ of free base); δ ($D_2O$, 360MHz) 0.94 (3H, t, J = 7.2Hz, $CH_3$), 1.74 (2H, sextet, J = 7.2Hz, $CH_2CH_2CH_3$), 1.97-2.06 (1H, m, 5-CH), 2.23-2.34 (1H, m, 5-CH), 2.75 (2H, t, J = 7.2Hz, $CH_2CH_2CH_3$), 3.31-3.45 (4H, m), 3.51-3.60 (1H, m) and 3.76-3.86 (3H, m, 2-$CH_2$, 3-CH, 4-CH, 6-$CH_2$ and 7-$CH_2$).

## EXAMPLE 23

endo-3-[5-(3-n-Propyl-1,2,4-oxadiazol)-yl]-1-azabicyclo[2.2.1]heptane hydrochloride

This was obtained from the reaction mixture during the preparation of the exo isomer, Example 22. The hydrochloride salt had mp 181.4-183.2°C. (Found: C, 54.11; H, 7.36; N, 17.00. $C_{11}H_{17}N_3O$. HCl requires C, 54.21; H, 7.44; N, 17.24%); m/e 207 ($M^+$ of free base); δ ($D_2O$, 360MHz) 0.94 (3H, t, J = 7.2Hz, $CH_3$), 1.58-1.66 (1H, m, 5-CH), 1.75 (2H, sextet, J = 7.2Hz, $CH_2CH_2CH_3$), 2.06-2.17 (1H, m, 5-CH), 2.77 (2H, t, J = 7.2Hz, $CH_2CH_2CH_3$), 3.33-3.60 (5H, m), 3.68-3.74 (1H, m), 3.92-4.00 (1H, m) and 4.15-4.20 (1H, m, 2-$CH_2$, 3-CH, 4-CH, 6-$CH_2$ and 7-$CH_2$).

## EXAMPLE 24

endo-3-[5-(3-Cyclopropyl-1,2,4-oxadiazol)yl]-1-azabicyclo[2.2.1]heptane hydrochloride

A freshly prepared solution of lithium diisopropylamide (prepared from 1.98ml of a 1.6M solution of n-butyllithium and diisopropylamine (0.23ml, 3.17mmol) in anhydrous tetrahydrofuran (10ml), cooled to -50°C) was cooled to -78°C and added dropwise to a cooled (-78°C), stirred solution of exo-3-[5-(3-cyclopropyl-1,2,4-oxadiazol)yl]-1-azabicyclo[2.2.1]heptane (0.26g, 1.26mmol, Example 5) in anhydrous tetrahydrofuran (50ml), under nitrogen. After stirring at -78°C for 2 hours the reaction mixture was quenched with a cooled (-78°C) solution of glacial acetic acid (0.19g, 3.17mmol) in anhydrous tetrahydrofuran (10ml). The reaction mixture was stirred whilst warming to room temperature and stirred for a further 0.5 hour. Water (20ml) was

added, basified to pH 10 with potassium carbonate and then extracted with dichloromethane (100ml). The organics were separated and the aqueous re-extracted with dichloromethane (4 x 100ml). The combined organics were dried (sodium sulphate) then evaporated to dryness and the residue purified by column chromatography on silica using dichloromethane/methanol (95:5) to afford the endo isomer (70mg). The hydrochloride salt had mp 178.7-180°C (propan-2-ol); Rf = 0.25 in dichloromethane/methanol (9:1) on silica. (Found: C, 53.94; H, 6.64; N, 17.09. $C_{11}H_{15}N_3O.HCl.0.1H_2O$ requires C, 54.25; H, 6.71; N, 17.26%); m/e 205 ($M^+$ of free base); δ ($D_2O$, 360MHz) 0.98-1.03 (2H, m, cyclopropyl $CH_2$), 1.13-1.19 (2H, m, cyclopropyl $CH_2$), 1.58-1.66 (1H, m, 5-CH), 2.04-2.18 (2H, m, cyclopropyl CH and 5-CH), 3.35-3.55 (5H, m), 3.68-3.74 (1H, m), 3.88-3.96 (1H, m) and 4.09-4.15 (1H, m, 2-$CH_2$, 3-CH, 4-CH, 6-$CH_2$ and 7-$CH_2$).

## EXAMPLE 25

exo-3-[5-(3-Isopropyl-1,2,4-oxadiazol)-yl]-1-azabicyclo[2.2.1]heptane hydrochloride

Prepared from 3-methoxycarbonyl-1-azabicyclo[2.2.1]heptane and isopropyl carboxamide oxime as described in Example 1. The hydrochloride salt had mp 122-125.9°C (ethyl acetate). (Found: C, 51.82; H, 7.44; N, 16.07. $C_{11}H_{17}N_3O.HCl.0.7H_2O$ requires C, 51.54; H, 7.63; N, 16.39%); m/e 207 ($M^+$ of free base); δ ($D_2O$, 360MHz) 1.31 (6H, d, J = 7Hz, 2 x $CH_3$), 1.98-2.06 (1H, m, 5-CH), 2.24-2.34 (1H, m, 5-CH), 3.13 (1H, septet, J = 7Hz, isopropyl CH), 3.31-3.47 (4H, m), 3.51-3.60 (1H, m) and 3.75-3.87 (3H, m, 2-$CH_2$, 3-CH, 4-CH, 6-$CH_2$ and 7-$CH_2$).

## EXAMPLE 26

endo-3-[5-(3-Isopropyl-1,2,4-oxadiazol)-yl]-1-azabicyclo[2.2.1]heptane hydrochloride

The title compound free base was obtained (110mg, 48%) from the exo isomer as described in Example 24. The hydrochloride salt had mp 196.3-196.5°C (propan-2-ol/diethyl ether). (Found: C, 54.07; H, 7.36; N, 17.23. $C_{11}H_{17}N_3O.HCl$ requires C, 54.21; H, 7.44; N, 17.24%); m/e 207 ($M^+$ of free base); δ ($D_2O$, 360MHz) 1.32 (6H, d, J = 7Hz, 2 x $CH_3$), 1.58-1.67 (1H, m, 5-CH), 2.07-2.16 (1H, m, 5-CH), 3.15 (1H, septet, J = 7Hz, isopropyl CH), 3.36-3.58 (5H, m, 4-CH, 6-$CH_2$ and 7-$CH_2$), 3.70-3.76 (1H, m, 2-CH), 3.92-4.00 (1H, m, 2-CH), 4.14-4.20 (1H, m, 3-CH).

## EXAMPLE 27

exo-3-[5-(3-(1-phenyl)ethyl-1,2,4-oxadiazol)-yl]-1-azabicyclo[2.2.1]heptane sesquioxalate and endo-3-[5-(3-(1-phenyl)ethyl-1,2,4-oxadiazol)yl]-1-azabicyclo[2.2.1]heptane hydrogen oxalate

Prepared from 3-methoxycarbonyl-1-azabicyclo[2.2.1]heptane (465mg, 3mmol) with 2-phenyl propionamide oxime (492mg, 3mmol) by the same method as Example 1. Column chromatography on neutral alumina (grade III) using dichloromethane/petroleum ether (7:3) yielded as the less polar fraction exo-3-[5-(3-(1-phenyl)ethyl-1,2,4-oxadiazol) yl]-1-azabicyclo[2.2.1]heptane (268mg).

This was treated with etheral oxalic acid to give the sesquioxalate as a hygroscopic solid. (Found: C, 55.5; H, 5.5; N, 10.0. $C_{16}H_{19}N_30.1.5(COOH)_2$ 0.5 $H_20$ requires C, 55.2; H, 5.6; N, 10.2%); m/e 270 $(M+H)^+$ of free base; δ ($D_20$, 360MHz) 1.66 (3H, d, J = 7Hz, $CH_3$), 1.90 - 2.04 (1H, m, 5-CH), 2.20 - 2.32 (1H, m, 5-CH), 3.22 - 3.44 (4H, m, 2x $NCH_2$), 3.45 - 3.56 (1H, m, 4-CH), 3.71 - 3.76 (3H, m, 3-CH and $NCH_2$), 4.36 (1H, q, J = 7Hz, $CHCH_3$), 7.32 - 7.44 (5H, m, $C_6H_5$).

The more polar fraction yielded endo-3-[5-(3-(1-phenyl)ethyl-1,2,4-oxadiazol)-yl]-1-azabicyclo[2.2.1]heptane (49.1mg). This was treated with ethereal oxalic acid to give the hydrogen oxalate salt as a solid, mp 158-159°C, (Found: C, 59.6; H, 6.0; N, 11.4 $C_{16}H_{19}N_3O$ $(COOH)_2$ 0.25$H_20$ requires C, 59.4; H, 6.0; N, 11.5%); m/e 270 $(M+H)^+$ of free base; δ ($D_20$, 360MHz) 1.44 - 1.60 (m, 1H, 5-CH), 1.67 (3H, d, J = 7Hz, $CH_3$), 1.98 - 2.14 (1H, m, 5-CH), 3.25 -3.95 (7H, m, 3x$NCH_2$ and 4-CH), 4.04 - 4.18 (1H, m, 3-CH), 4.36 (1H, q, J = 7Hz,C<u>H</u>$CH_3$), 7.32 - 7.43 (5H, m, $C_6H_5$).

## EXAMPLE 28

3-[5-(3-(1,1-Diphenylmethyl)-1,2,4-oxadiazol)-yl]-quinuclidine dihydrogen oxalate

This was prepared from 3-methoxycarbonyl quinuclidine (0.820g, 4.9mmol) with 1,1-diphenylmethyl carboxamide oxime (1.08g, 4.8mmol) by the same method as Example 1. Column chromatography on neutral alumina (grade III) using dichloromethane gave an oil which was treated with excess ethereal oxalic acid and

the resulting precipitate crystallised from methanol/diethylether to yield the title product (1.06g), mp 149-150°C, (Found: C, 59.4; H, 5.2; N, 8.0. $C_{22}H_{23}N_3O$. 2 $(COOH)_2$ requires C, 59.4; H, 5.2; N, 8.0%); m/e 346 $(M+H)^+$ of free base; δ ($D_2O$, 360MHz) 1.72-1.76 (1H, m) and 1.84-1.90 (1H, m) and 2.04-2.20 (2H, m, 5-$CH_2$ and 8-$CH_2$), 2.60-2.61 (1H, m, 4-CH), 3.32-3.40 (4H, m, 6-$CH_2$ and 7-$CH_2$), 3.76-3.90 (3H, m, 2-$CH_2$ and 3-CH), 5.75 (1H, s, CHPh), 7.32-7.43 (10H, m, $2xC_6H_5$).

## EXAMPLE 29

3-[5-(3-(1-Hydroxy-1-phenylpropyl)-1,2,4-oxadiazol)-yl]-quinuclidine hydrogen oxalate

This was prepared from 3-[3-Benzoyl-1,2,4-oxadiazol-5-yl)-quinuclidine (650mg, 2.3mmol) and ethyl magnesium bromide (3.2ml of a 1.4M solution in ether, 4.5mmol) by the same method as Example 12. Column chromatography on neutral alumina yielded an oil (263mg) which was treated with excess ethereal oxalic acid solution to afford the title compound as a solid, mp 59-60°C, (Found: C, 56.9; H, 6.2; N, 9.7. $C_{18}H_{23}N_3O_2.(COOH)_2.H_2O$ requires C, 57.0; H, 6.5; N, 10.0%); m/e 314 $(M+H)^+$ of free base, δ ($D_2O$, 360MHz) 0.88 (3H, t, J = 7Hz, $CH_2CH_3$), 1.60 - 1.75 (1H, m) and 1.80 -1.95 (1H, m) and 2.10 -2.13 (2H, m, 5-$CH_2$ and 8-$CH_2$), 2.31 - 2.44 (2H, m, $CH_2CH_3$), 2.60 - 2.62 (1H, m, 4-CH), 3.31 -3.47 (4H, m, 6-$CH_2$ and 7-$CH_2$), 3.75 - 3.89 (3H, m, 2-$CH_2$ and 3-CH), 7.36 - 7.66 (5H, m, $C_6H_5$).

## EXAMPLE 30

3-[5-(3-(1-Hydroxy-1-phenylprop-2-enyl)-1,2,4-oxadiazol)-yl] quinuclidine sesquioxalate

This was prepared from 3-(3-benzoyl-1,2,4-oxadiazol-5-yl)-quinuclidine (650mg, 2.3mmol) and vinyl magnesium bromide (4.6ml of a 1M solution in tetrahydrofuran) by the same method as Example 12. Column chromatography on neutral alumina yielded an oil (254mg) which was treated with excess ethereal oxalic acid solution to afford the title compound as a solid, mp 62-65°C, (Found: C, 56.2; H, 5.6; N, 9.2. $C_{18}H_{21}N_3O_2$.1.5 $(COOH)_2$ requires C, 56.5; H, 5.4; N, 9.4%), m/e 312 $(M+H)^+$ of free base, δ ($D_2O$, 360MHz) 1.76-1.82 (1H, m) and 1.84-1.98 (1H, m) and 2.04-2.20 (2H, m, 5-$CH_2$ and 8-$CH_2$), 2.62-2.63 (1H, m, 4-CH), 3.35-3.42 (4H, m, 6-$CH_2$ and 7-$CH_2$) 3.76-3.91 (3H, m, 2-$CH_2$ and 3-CH), 5.29-5.41 (2H, m, $CH_2=CH$), 6.45 (1H, dd, J = 10 and 17Hz, $CH_2=CH$), 7.41-7.46 (5H, m, $C_6H_5$).

## EXAMPLE 31

3-[5-(3-(1-Hydroxy-1-(2-naphthyl)-1-phenyl)methyl-1,2,4-oxadiazol)-yl]quinuclidine dihydrogen oxalate

This was prepared from 3-(3-benzoyl-1,2,4-oxadiazol-5-yl)-quinuclidine (283mg, 1mmol) and the Grignard reagent formed between 2-bromonaphthalene (207mg, 1mmol) and magnesium (24mg, 1mmol) by the same method as Example 12. Column chromatography on neutral alumina (grade III) using dichloromethane gave an oil which was treated with excess ethereal oxalic acid to give a solid, mp 55-56°C, (Found: C, 60.9; H, 5.1; N, 7.4.$C_{26}H_{25}N_3O_2$.2$(COOH)_2$ requires C, 60.9; H, 4.9; N, 7.1%); m/e 412 $(M+H)^+$ of free base, δ ($D_2O$, 360MHz) 1.67-1.80 (1H, m) and 1.81-1.96 (1H, m) and 2.04 -2.22 (2H, m, 5-$CH_2$ and 8$CH_2$), 2.61-2.68 (1H, m, 4-CH), 3.27-3.45 (4H, m, 6-$CH_2$ and 7-$CH_2$), 3.72-3.98 (3H, m, 2-$CH_2$ and 3-CH), 7.38-7.98 (12H, m, $C_6H_5$ and $C_{10}H_7$).

## EXAMPLE 32

3-[5-(3-(1-Hydroxy-1-(4-methoxyphenyl)-1-phenyl)methyl-1,2,4-oxadiazol)-yl]quinuclidine sesquioxalate

This was prepared from 3-(3-benzoyl-1,2,4- oxadiazol-5-yl)-quinuclidine (566mg, 2mmol) and the Grignard reagent formed between 4-bromoanisole (374mg, 2mmol) and magnesium (48.6mg, 2mmol) by the same method as Example 12. Column chromatography on neutral alumina (Grade III) using dichloromethane gave an oil which was treated with excess ethereal oxalic acid to give a solid (175mg), mp 43-45°C, (Found: C, 55.8; H, 5.5; N, 7.4.$C_{23}H_{25}N_3O_3$ 1.5 $(COOH)_2.2H_2O$ requires C, 55.5; H, 5.7; N, 7.5%); m/e 392 $(M+H)^+$ of free base; δ ($D_2O$, 360MHz) 1.64- 2.28 (4H, m, 5-$CH_2$ and 8-$CH_2$), 2.60-2.64 (1H, m, 4-CH), 3.28-4.03 (7H, m, 2-$CH_2$, 7-$CH_2$, 6$CH_2$ and 3-CH), 3.83 (3H, s, $OCH_3$), 6.98-7.44 (9H, m, $C_6H_5$ and $C_6H_4(OCH_3)$).

## EXAMPLE 33

3-[5-(3-(1-(4-Fluorophenyl)-1-hydroxy-1-phenyl)methyl-1,2,4-oxadiazol)-yl] quinuclidine sesquioxalate

This was prepared from 3-(3-benzoyl-1,2,4-oxadiazol-5-yl)-quinuclidine (566mg, 2mmol) and the Grignard reagent formed between 1-bromo-4-fluorobenzene (350mg, 2mmol) and magnesium (48.6mg, 2mmol) by the same method as Example 12. Column chromatography on neutral alumina (grade III) using dichloromethane yielded an oil which was treated with excess ethereal oxalic acid to afford the title compound as a solid (235mg), mp 55-56°C. (Found: C, 56.4; H, 5.1; N, 7.9, $C_{22}H_{22}N_3O_2F$ 1.5 $(COOH)_2.H_2O$ requires C, 56.5; H, 5.1; N, 7.8%); m/e 380 $(M+H)^+$ of free base, δ ($D_2O$, 360MHz) 1.64-1.78 (1H, m) and 1.82-1.96 (1H, m) and 2.04-2.24 (2H, m, 5-$CH_2$ and 8-$CH_2$), 2.62-2.65 (1H, m, 4-CH), 3.32-3.43 (4H, m, 6-$CH_2$ and 7-$CH_2$), 3.74-3.82 (2H, m, $2CH_2$), 3.90-3.93 (1H, m, 3-CH), 7.12-7.45 (9H, m, $C_6H_4F$ and $C_6H_5$).

EXAMPLE 34

exo-3-[5-(3-(1,1-Diphenyl-1-hydroxy)-methyl-1,2,4-oxadiazol)-yl]-1-azabicyclo[2.2.1]heptane hydrogen oxalate

a) 2,2-Diphenyl-2-tetrahydropyranyloxyacetamide oxime

This was prepared from 2,2-Diphenyl-2-tetrahydropyranyloxy-acetonitrile (5.14g, 20mmol) with hydroxylamine hydrochloride (2.1g, 30mmol) and sodium (690mg, 30mmol) in the same manner as Example 9a. Column chromatography on silica and crystallisation from petrol (40-60°C) yielded product (2.9g), mp 130-132°C. (Found: C, 69.9; H, 6.8; N, 8.4. $C_{19}H_{22}N_2O_3$ requires C, 69.9; H, 6.8; N, 8.6%); δ (D6-DMSO, 360MHz) 1.40-1.83 (6H, m, 3 x $CH_2$), 3.16-3.18 and 3.80-3.85 (each 1H, each m, $CH_2O$), 4.58 (1H, t, J = 4Hz, CHO), 5.25 (2H, bs, $NH_2$), 7.21-7.50 (10H, m, 2 x $C_6H_5$), 9.36 (1H, s, OH).

b) exo-3-[5-(3-(1,1-Diphenyl-1-tetrahydropyranoxy)-methyl-1,2,4-oxadiazol)-yl]-1-azabicyclo[2.2.1]heptane

This was prepared from 3-methoxycarbonyl-1-azabicyclo[2.2.1]heptane (465mg, 3mmol) and 2,2-diphenyl-2-tetrahydropyranyloxy-acetamide oxime (978mg, 3mmol) using the method of Example 1. Column chromatography on silica using dichloromethane/petrol (7:3) yielded 535mg as an oil. (Found: C, 71.9; H, 6.9; N, 9.3. $C_{26}H_{29}N_3O_3$.0.25 ($H_2O$) requires C, 71.6; H, 6.8; N, 9.6%), (Found: $M^+$ 431.2194 $C_{26}H_{29}N_3O_3$ requires 431.2200); δ ($CDCl_3$, 360MHz) 1.06-1.68 (6H, m, 3 x $CH_2$), 1.68-1.88 (1H, m, 5-CH), 1.72-2.07 (1H, m, 5-CH), 2.38-2.46 (1H, m) and 2.47-2.58 (1H, m) and 2.76-3.18 (7H, m, 4-CH, 2-$CH_2$, 6-$CH_2$, 7-$CH_2$, $OCH_2$) 3.74-3.85 (1H, m, 3-CH), 4.98 (1H, t, J = 3Hz, CHO), 7.22-7.64 (10H, m, 2 x $C_6H_5$).

c) exo-3-[5-(3-(1,1-Diphenyl-1-hydroxy)-methyl-1,2,4-oxadiazol)-yl]-1-azabicyclo[2.2.1]heptane hydrogen oxalate

This was prepared from exo-3-[5-(3-(1,1-diphenyl-1-tetrahydropyranyloxy)-methyl-1,2,4-oxadiazol)-yl]-azabicyclo[2.2.1]heptane (500mg) in the same way as Example 9c to give an oil. This was precipitated with excess ethereal oxalic acid solution to give a solid, mp 189-190°C. (Found: C, 62.8; H, 5.3; N, 9.6. $C_{21}H_{21}N_3O_2.(COOH)_2$ requires C, 63.1; H, 5.3; N, 9.6%); δ ($D_2O$, 360MHz) 1.94-2.04 (1H, m, 5-CH), 2.18-2.32 (1H, m, 5-CH), 3.24-3.44 (4H, m, 2 x $NCH_2$), 3.44-3.58 (1H, m 4-CH), 4.72-4.86 (3H, m, 3-CH and $NCH_2$), 7.35-7.44 (10H, m, 2 x $C_6H_5$).

EXAMPLE 35

3-[5-(3-(1-Hydroxy-1-(2-pyridyl))-methyl-1,2,4-oxadiazol)-yl]-quinuclidine

a) 2-(2-pyridyl)-2-tetrahydropyranyloxyacetamide oxime

This was prepared from 2-(2-pyridyl)-2-tetrahydropyranyloxy acetonitrile (12.6g, 0.058mol) with hydroxylamine hydrochloride (4.0g, 0.058mol) and sodium (1.33g, 0.058mol) in the same manner as Example 9a. Column chromatography on silica using dichloromethane/methanol (95:5) followed by crystallisation from ethyl acetate/petrol (40-60) yielded a white solid, (6.4g), mp 149-150°C. (Found: C, 57.2; H, 6.8; N, 16.6. $C_{12}H_{17}N_3O_3$ requires C, 57.4; H, 6.8; N, 16.7%); m/e 252 $(M+H)^+$; δ (D-6, DMSO, 360MHz) 1.22-1.90 (6H, m, 3 x $CH_2$), 3.36-3.50 and 3.58-3.72 and 3.80-3.86 (2H, m, $CH_2O$), 4.49 (0.7H, t, J = 5Hz) and 4.77 (0.3H, t, J = 5Hz, CHO), 5.04 (0.3H, s) and 5.10 (0.7H, s, $C_5H_4NCH$), 5.27 (2H, bs, $NH_2$), 7.28-7.33 (1H, m, pyridine 5-CH), 7.51-7.70 (1H, m, pyridine 3-CH), 7.76-7.83 (1H, m, pyridine 4-CH), 8.51-8.52 (1H, m, pyridine 6-CH), 9.20 (0.7H, s, OH), 9.24 (0.3H, s, OH).

b) 3-[5-(3-1-(2-pyridyl)-1-tetrahydropyranyloxy)-methyl-1,2,4-oxadiazol)-yl]quinuclidine

This was prepared from 2-(2-pyridyl)-2-tetrahydropyranyloxy-acetamide oxime (3.3g, 9mmol) in the same way as Example 9c to give product as an oil, (3.3g), Rf = 0.2 on alumina using dichloromethane/methanol (99:1). (Found: $M^+$ 370.2033, $C_{20}H_{26}N_4O_3$ requires $M^+$ 370.2005); δ ($CDCl_3$, 250MHz) 1.26-2.18 (10H, m, 5-$CH_2$

and 8-$CH_2$ and 3 x $CH_2$), 2.20-2.23 (1H, m, 4-CH), 3.10-3.62 (8H, 6-$CH_2$, 7-$CH_2$, 2-$CH_2$ and $OCH_2$), 3.66-4.04 (1H, m, 3-CH), 4.83 (0.5H, t, J = 3Hz) and 4.91 (0.5H, t, J = 3Hz, CHO), 6.09 and 6.13 (each 0.5H, each s, $C_5H_4NCH$), 7.22-7.26 (1H, m, pyridine 5-CH), 7.68-7.79 (2H, m, pyridine 3-CH and 4-CH), 8.53-8.58 (1H, m, pyridine 6CH).

c) 3-[5-(3-(1-Hydroxy-1-(2-pyridyl))-methyl-1,2,4-oxadiazol)-yl]quinuclidine

This was prepared from 3-[5-(3-(1-(2-pyridyl)-1-tetrahydropyranyloxy)-methyl-1,2,4-oxadiazol)-yl] quinuclidine (3.3g, 9mmol) in the same way as Example 9c to give an oil (2.5g). (Found: $M^+$ 286.1443 $C_{15}H_{18}N_4O_2$ requires $M^+$ 286.1430); δ ($CDCl_3$, 250MHz) 1.32-1.46 (1H, m) and 1.50-1.60 (1H, m) and 1.62-1.72 (2H, m, 5-$CH_2$ and 8-$CH_2$), 2.20-2.23 (1H, m, 4-CH), 2.78-2.97 (6H, m, 2-$CH_2$, 6-$CH_2$, 7-$CH_2$), 3.14-3.42 (1H, m, 3-CH), 5.98 (1H, s, $C_5H_4NCH$), 7.28 (1H, ddd, J = 2,5 and 8Hz, pyridyl 4-CH), 7.46 (1H, dd, J = 2 and 8Hz, pyridyl 3-CH), 7.74 (1H, dt, J = 2 and 8Hz, pyridyl 5-CH), 8.62 (1H, d, J = 5Hz, pyridyl 6-CH).

## EXAMPLE 36

### 3-[5-(3-(1-Hydroxy-1-phenyl-1-(2-pyridyl))-methyl-1,2,4-oxadiazol)-yl]quinuclidine dihydrogen oxalate

a) 3-[5-(3-(2-Pyridoyl)-1,2,4-oxadiazol)-yl]quinuclidine

This was prepared from 3-[5-(3-(1-Hydroxy-1-(2-pyridyl))-methyl-1,2,4-oxadiazol)-yl]quinuclidine (2.0g, 6.9mmol) and manganese dioxide (5.0g) in the same way as Example 11 to yield 1.6g of an oil, Rf = 0.1 on alumina using ethyl acetate/methanol (99:1); $\nu_{max}$ (liquid film) 1695$cm^{-1}$; δ ($CDCl_3$, 250MHz) 1.40-1.53 (1H, m) and 1.63-1.88 (3H, m, 5-$CH_2$ and 8-$CH_2$, 2.31-2.35 (1H, m, 4-CH), 2.82-3.12 (4H, m) and 3.32-3.60 (3H, m, 2-$CH_2$, 6-$CH_2$, 7-$CH_2$ and 3-CH), 7.59 (1H, ddd, J = 2,5 and 8Hz, pyridyl 5-CH), 7.95 (1H, dt, J = 2 and 8Hz, pyridyl 4-CH), 8.24 (1H, dt, J = 1 and 8Hz, pyridyl 3-CH), 8.84 (1H, ddd, J = 1,2 and 8Hz, pyridyl 6-CH).

b) 3-[5-(3-(1-Hydroxy-1-phenyl-1-(2-pyridyl))methyl-1,2,4-oxadiazol)-yl]quinuclidine dioxalate dihydrogen oxalate

This was prepared from 3-[5-(3-(2-pyridoyl)-1,2,4-oxadiazol)-yl]quinuclidine (1.0g, 3.5mmol) and phenyl magnesium bromide (3M solution in THF, 5ml, 15mmol) in the same way as Example 12 to yield the title product as the dihydrogen oxalate salt (455mg), mp 40-42°C. (Found: C, 51.8; H, 4.9; N, 9.4. $C_{21}H_{22}N_4O_2$. $2(COOH)_2$. $2H_2O$ requires C, 51.9; H, 5.2; N, 9.7%); δ ($D_2O$, 360MHz) 1.62-2.24 (4H, m, 5-$CH_2$ and 8-$CH_2$), 2.52-2.72 (1H, m, 4-CH), 3.24-3.56 (4H, m, 6-$CH_2$ and 7-$CH_2$), 3.76-3.84 (2H, m, 2-$CH_2$), 3.98-4.04 (1H, m, 3-CH), 7.41-7.51 (5H, m, $C_6H_5$), 8.03-8.50 (2H, m, pyridyl 3-CH and 4-CH), 8.50-8.60 (1H, m, pyridyl 5-CH), 8.68-8.92 (1H, m, pyridyl 6-CH).

## EXAMPLE 37

### 1R*,6R* and 1R*,6S*-6-[5-(3-Cyclopropyl-1,2,4-oxidiazol)-yl]-2-azabicyclo[2.2.2]octane hydrogen oxalate

a) Methyl 2-t-butyloxycarbonyl-2-azabicyclo[2.2.2]octane-6-carboxylate

Di-t-butyldicarbonate (21.8g, 0.10mol) in dry dichloromethane (50ml) was added dropwise to a stirred, cooled (0°C) solution of methyl 2-azabicyclo[2.2.2]octane-6-carboxylate (18.2g, 0.09mol, mixture of endo and exo isomers, prepared as described in Example 21a, EP 0239309) in dry dichloromethane (100ml). The resulting solution was stirred at room temperature for 4 hours, water (100ml) was added and the mixture was stirred for 15 minutes. The organic layer was separated and washed with 0.5M hydrochloric acid (100ml), water (100ml), saturated sodium hydrogen carbonate solution (100ml), water (100ml) then dried (sodium sulphate) and evaporated to dryness. The residue was purified by column chromatography on silica by elution with ethyl acetate/petroleum ether (60-80) [1:40] to give Isomer A as a colourless oil which crystallised on standing (12.0g), mp 44-45°C, Rf = 0.35 in ethyl acetate/petroleum ether (60-80) [1:1] on silica. (Found: C, 62.59, H, 8.55; N, 5.10. $C_{14}H_{23}NO_4$ requires C, 62.43; H, 8.61; N, 5.20%); m/e 269 ($M^+$); $\nu_{max}$ (film) 1740 and 1695$cm^{-1}$ (C=O); δ ($CDCl_3$, 360MHz) 1.47 (9H, s, $C(CH_3)_3$); 1.55-2.20 (7H, m, 4-CH, 5-$CH_2$, 7-$CH_2$ and 8-$CH_2$); 2.86-3.00 (1H, m, 6-CH); 3.30 (2H, broad s, 3-$CH_2$); 3.69 and 3.72 (total 3H, each broad s, $CO_2CH_3$, rotamers); 4.21 and 4.38 (total 1H, each broad s, 1-CH, rotamers). Mixed fractions were collected (1:1 mixture, 4.80g) followed by Isomer B as a colourless oil (6.80g), Rf = 0.32 in ethyl acetate/petroleum ether (60-80) [1:1] on silica; m/e 269 ($M^+$); δ ($CDCl_3$, 360MHz) 1.42 and 1.43 (total 9H, each s, $C(CH_3)_3$, rotamers); 1.52-2.20 (7H, m, 4-CH, 5-$CH_2$, 7-$CH_2$ and 8-$CH_2$); 2.63-2.73 (1H, m, 6-CH); 3.19-3.25 (1H, m, 3-CH); 3.36-3.42 (1H, m, 3-CH); 3.66 and 3.69 (total 3H, each s, $CO_2CH_3$, rotamers); 4.27-4.30 and 4.36-4.38 (total 1H, each m, 1-CH, rotamers).

b) 2-t-Butoxycarbonyl-6-[5-(3-cyclopropyl-1,2,4-oxadiazol)-yl]-2-azabicyclo[2.2.2]octane

Sodium hydride (1.82g of a 55% dispersion in oil, 41.7mmol) was added to a stirred suspension of cyclopropane carboxamide oxime (3.86g, 38.6mmol) in dry tetrahydrofuran (135ml) containing 4A molecular sieves (4g), and the mixture heated at 50°C for 1.5h. A solution of the foregoing ester, as a mixture of diastereoisomers, (5.0g, 18.6mmol) in tetrahydrofuran (20ml) was added and the mixture heated under reflux for 3h. Water (50ml) was added to the cooled reaction mixture and the product extracted into dichloromethane (4 x 50ml). The combined extracts were dried (magnesium sulphate), the solvents evaporated and the residue purified by column chromatography on silica in dichloromethane/ethyl acetate (98:2) to give pure isomer A, (1.2g) which crystallised on standing, mixed fractions (1.32g) and pure isomer B (2.58g) as an oil. Isomer A is the 1R*,6R* (anti) isomer m.p. 78-79°C, m/e 319.1877 ($M^+$), $C_{17}H_{25}N_3O_3$ requires m/e 319.1896; δ ($CDCl_3$, 360MHz) 0.98-1.06 (4H, m, cyclopropyl $C\underline{H_2}C\underline{H_2}$); 1.48 and 1.58 (total 9H, each s, $C(CH_3)_3$, rotamers); 1.50-1.80 (5H, m, 7-$CH_2$ and 8-$CH_2$ and cyclopropyl $C\underline{H}$); 2.00-2.15 (4H, m, 4-CH, 5-$CH_2$ and 6-CH); 3.36-3.48 (2H, m, 3-$CH_2$); 4.25 and 4.42 (total 1H, each broad s, 1-CH, rotamers). Isomer B is the 1R*,6S* (syn) isomer, m/e 319.1882 ($M^+$); $C_{17}H_{25}N_3O_3$ requires m/e 319.1896; δ ($CDCl_3$, 360MHz), 0.97-1.02 (4H, m, cyclopropyl $C\underline{H_2}C\underline{H_2}$); 1.30 and 1.37 (total 9H, each s, $C(CH_3)_3$, rotamers); 1.58-1.76 (4H, m, 7-$CH_2$ and 8-$CH_2$); 1.94-2.28 (5H, m, 4-CH, 5-$CH_2$, 6-CH and cyclopropyl $C\underline{H}$); 3.21-3.30 and 3.48-3.53 (total 2H, m, 3-$CH_2$, rotamers); 4.20-4.23 and 4.29-4.31 (total 1H, m, 1-CH, rotamers).

c) 1R*,6R*-6-[5-(3-Cyclopropyl-1,2,4-oxadiazol)-yl]-2-azabicyclo[2.2.2]octane hydrogen oxalate

To a ice-cooled solution of the foregoing t-butoxycarbonyl amine, isomer A, (1.18g, 3.70mmol) in dichloromethane (15ml) was added trifluoracetic acid (5.88ml, 76.4mmol). The ice bath was removed and the solution stirred at room temperature for 3h. Aqueous potassium carbonate (2M, 30ml) was added and the product extracted with dichloromethane (4 x 50ml). The combined organic layers were dried (sodium sulphate) and evaporated to yield the crude product (0.81g, 100%), which was converted to the hydrogen oxalate salt and recrystallised from ethanol m.p. 180-185°C. (Found: C, 53.96; H, 6.20; N, 13.25. $C_{12}H_{17}N_3O.C_2H_2O_4$ requires C, 54.36; H, 6.19; N, 13.58%); m/e 219 ($M^+$ of free base); δ ($D_2O$, 360MHz) 0.95-0.98 and 1.10-1.16 (each 2H, each m, cyclopropyl $CH_2CH_2$); 1.68-1.86 (3H, m, 7-$CH_2$ and 8-CH); 1.93-2.02 (1H, m, 8-CH); 2.06-2.21 (3H, m, 4-CH, 5-CH and cyclopropyl $C\underline{H}CH_2$); 2.27-2.35 (1H, m, 5-CH); 3.32 (2H, broad s, 3-$CH_2$); 3.75-3.81 (1H, m, 6-CH); 3.88 (1H, broad s, 1-CH).

d) 1R*,6S*-6-[5-(3-Cyclopropyl-1,2,4-oxadiazol)-yl]-2-azabicyclo[2.2.2]octane hydrogen oxalate

The t-butoxycarbonyl amine, isomer B, (453mg, 1.42mmol) was treated as above to yield the deprotected amine free base, which was purified by column chromatography on alumina in dichloromethane/methanol (99:1) (236mg, 76%), which was converted to the hydrogen oxalate salt and recrystallised from ethanol (recovery 272mg), m.p. 129-131°C (Found: C, 52.86; H, 6.01; N, 12.89. $C_{12}H_{17}N_3O$. 1.2 $C_2H_2O_4$ requires: C, 52.84; H, 5.97; N, 12.84%); m/e 219 ($M^+$ of free base); δ ($D_2O$, 360MHz) 0.96-1.01 and 1.10-1.16 (each 2H, each m, cyclopropyl $CH_2CH_2$); 1.78-1.86 (2H, m, 8-$CH_2$); 1.96-2.19 (5H, m, 4-CH, 5-CH, 7-$CH_2$ and cyclopropyl $C\underline{H}$); 2.38 (1H, dt, J = 1Hz and 12Hz, 5-CH); 3.28 (2H, s, 3-$CH_2$); 3.67 (1H, ddd, J = 1Hz, 6Hz and 12Hz, 6-CH); 3.94 (1H, broad, s, 1-CH).

## EXAMPLE 38

### 1R*,6R* and 1R*,6S*-6-[5-(3-Isopropyl-1,2,4-oxadiazol)-yl]-2-azabicyclo[2.2.2]octane hydrogen oxalate

a) 2-t-Butoxycarbonyl-6-[5-(3-Isopropyl-1,2,4-oxadiazol)-yl]-2-azabicyclo[2.2.2]octane

Prepared as described in Example 37 using isobutyramide oxime to yield isomer A, the 1R*,6R* (anti) isomer, m/e 321.2047 ($M^+$); $C_{17}H_{27}N_3O_3$ requires m/e 321.2052; δ ($CDCl_3$, 360MHz) 1.31-1.35 (6H, overlapping doublets, $CH(C\underline{H_3})_2$, rotamers); 1.48 (9H, s, $C(CH_3)_3$; 1.54-1.78 (4H, m, 7-$CH_2$ and 8-$CH_2$); 2.02-2.25 (3H, m, 4-CH and 5-$CH_2$); 3.04-3.10 (1H, m, $C\underline{H}(CH_3)_2$, rotamers); 3.39 (2H, broad s, 3-$CH_2$); 3.42-3.54 (1H, m, 6-CH); 4.27 and 4.46 (total 1H, each broad s, 1-CH, rotamers); and isomer B, the 1R*,6S* (syn) isomer, m/e 321.2055 ($M^+$); $C_{17}H_{27}N_3O_3$ requires m/e 321.2052; δ ($CDCl_3$, 360MHz) 1.29 and 1.38 (total 9H, each s, $C(CH_3)_3$, rotamers) 1.29-1.34 (6H, overlapping d, $CH(C\underline{H_3})_2$, rotamers); 1.62-1.86 (4H, m, 7-$CH_2$ and 8-$CH_2$); 1.94-2.35 (3H, m, 4-CH and 5-$CH_2$); 2.99-3.09 (1H, m, $C\underline{H}(CH_3)_2$, rotamers); 3.26-3.38 (2H, m, 3-$CH_2$); 3.49-3.56 (1H, m, 6-CH); 4.29 and 4.34 (total 1H, each broad s, 1-CH, rotamers).

b) 1R*,6R*-6-[5-(3-Isopropyl-1,2,4-oxadiazol)-yl]-2-azabicyclo[2.2.2]octane hydrogen oxalate

The foregoing t-butyl carbamate, isomer A, (615mg, 1.92mmol) was treated as described in Example 37(c) to yield the deprotected amine free base (420mg, 99%), which was converted to the hydrogen oxalate salt and crystallised from ethanol (recovery 253mg) m.p. 160-161°C. (Found: C, 54.06; H, 6.82; N, 13.40. $C_{12}H_{19}N_3O.C_2H_2O_4$ requires: C, 54.01; H, 6.80; N, 13.50%); m/e 221 ($M^+$ of free base); δ ($D_2O$, 360MHz) 1.30 (6H, d, J = 7Hz, $CH(C\underline{H_3})_2$); 1.70-1.85 (3H, m, 7-CH and 8$CH_2$); 1.94-2.02 (1H, m, 7-CH); 2.16-2.25 (2H, m, 4-CH and 5-CH); 2.30-2.40 (1H, m, 5-CH); 3.12 (1H, septet, J = 7Hz, $C\underline{H}(CH_3)_2$); 3.34 (2H, s, 3-$CH_2$); 3.79-3.88

(1H, m, 6-CH); 3.91 (1H, broad s, 1-CH).

C) 1R*,6S*-6-[5-(3-Isopropyl-1,2,4-oxadiazol)-yl]-2-azabicyclo[2.2.2]octane hydrogen oxalate

The foregoing t-butyl carbamate, isomer B (872mg, 2.72mmol) was treated as above to yield the deprotected amine (571mg, 95%), which was converted to the hydrogen oxalate salt and crystallised from ethanol (recovery 350mg) m.p. 124-125°C. (Found: C, 53.85; H, 6.81; N, 13.34. $C_{12}H_{19}N_3O.C_2H_2O_4$ requires: C, 54.01; H, 6.80; N, 13.50%); m/e 221 ($M^+$ of free base); δ ($D_2O$, 360MHz) 1.30 (6H, d, J = 7Hz, CH($CH_3$)2); 1.80-1.88 (2H, m, 8-$CH_2$); 2.00-2.14 (3H, m, 5-CH and 7-$CH_2$); 2.17-2.20 (1H, m, 4-CH); 2.37-2.45 (1H, m, 5-CH); 3.12 (1H, septet, J = 7Hz, CH($CH_3$)2); 3.29 (2H, s, 3-$CH_2$); 3.71 (1H, ddd, J = 1Hz, 6Hz and 12Hz, 6-CH); 3.96-4.00 (1H, m, 1-CH).

## EXAMPLE 39

1R*, 6S* - 6-[5-(3-Cyclopropyl-1,2,4-oxadiazol)-yl]-2-methyl-2-azabicyclo[2.2.2]octane sesquioxalate and 1R*, 6R* - 6-[5-(3-cyclopropyl-1,2,4-oxadiazol)-yl]-2-methyl-2-azabicyclo[2.2.2]octane hydrogen oxalate

A stirred solution of 1R*, 6S* - 6-[5-(3-cyclopropyl-1,2,4-oxadiazol)-yl]-2-azabicyclo[2.2.2]octane from Example 37(d) (681mg, 3.11mmol) in aqueous formaldehyde (35%, 5ml) and formic acid (5ml) was heated under reflux for 1hour. The cooled solution was evaporated to dryness, and partitioned between aqueous potassium carbonate and dichloromethane. The organic layer was dried (potassium carbonate) and evaporated to yield a mixture of diastereoisomers which were separated by column chromatography on silica in dichloromethane/methanol (95:5) to yield isomer A (the 1R*, 6S* isomer) (241mg) and isomer B (the 1R*, 6R* isomer) 262mg. Isomer A was converted to the sesquioxalate and crystallised from ethanol (197mg) m.p. 134-135°C. (Found, C, 52.26; H, 6.10; N, 11.46. $C_{13}H_{19}H_30$ 1.5 $C_2H_20_4$ requires: C, 52.17; H, 6.02; N, 11.41%); m/e 233 ($M^+$ of free base); δ ($D_20$, 360MHz) 0.96-1.01 and 1.10-1.18 (each 2H, each m, cyclopropyl $CH_2CH_2$); 1.74-2.02 (4H, m, 7-$CH_2$ and 8 $CH_2$); 2.08-2.16 (1H, m, cyclopropyl CH); 2.18-2.22 (1H, m, 4-CH); 2.25-2.34 (1H, m, 5-CH); 2.41-2.53 (1H, m, 5-CH); 2.94 (3H, s, $NCH_3$); 2.99 (1H, d, J = 12Hz, 3-CH); 3.59 (1H, dt, J = 1Hz and 12Hz, 3-CH); 3.73 (1H, dd, J = 7Hz and 12Hz, 6-CH); 3.91 (1H, broad s, 1-CH). Isomer B was converted to the hydrogen oxalate and crystallised from ethanol (165mg) m.p. 127-128°C. (Found: C, 55.56; H, 6.53; N, 12.93. $C_{13}H_{19}N_30$. $C_2H_20_4$ requires: C, 55.72; H, 6.54; N, 13.00%); m/e 233 ($M^+$ of free base); δ ($D_20$, 360MHz) 0.95-1.00 and 1.10-1.16 (each 2H, each m, cyclopropyl $CH_2CH_2$); 1.62-1.96 (3H, m, 7-CH and 8-$CH_2$); 2.02-2.41 (5H, m, 4-CH, 5-$CH_2$, 7-CH and cyclopropyl CH); 3.01 (3H, s, $CH_3$); 3.05 (1H, d, J = 13Hz, 3-CH); 3.62-3.71 (1H, m, 6-CH); 3.76-3.81 (1H, m, 1-CH); 3.80-3.86 and 3.96-4.02 (total 1H, each m, 3-CH, invertomers at N).

## EXAMPLE 40

1R*, 6R* and 1R*, 6S* - 6-[5-(3-Cyclopropyl-1,2,4-oxadiazol)-yl]-2-azabicyclo[2.2.2]oct-7-ene hydrochloride

a) Methyl 2-t-Butyloxycarbonyl-2-azabicyclo[2.2.2]oct-7-ene-6-carboxylate

Methyl Z-benzyloxycarbonyl-2-azabicyclo[2.2.2]oct-7-ene-6-carboxylate (3.6g, 12.0mmol), mixture of exo and endo isomers (prepared as described in EP 0239309, Example 21A), was treated with hydrogen bromide in acetic acid (48%, 16ml) at 22°C for 30 min, the solution diluted with ether (150ml) and the supernatant discarded. Aqueous potassium carbonate (20ml) was added to the residue and extracted with dichloromethane (4 x 50ml). Evaporation of the combined organic layers yielded the deprotected amine (1.16g, 6.9mmol) which was immediately treated with di-t-butyldicarbonate (1.56g, 7.14mmol) in dichloromethane (4ml). After stirring for 4hours, the solvent was removed and the residue purified by column chromatography on silica in dichloromethane/ethyl acetate (95:5) to yield isomer A (532mg) and isomer B (178mg) along with mixed fractions (450mg). Isomer A (the 1R*, 6R* isomer) crystallised on standing, m.p. 107-108°C. (Found: C, 62-67; H, 7.90; N, 5.18. $C_{14}H_{21}NO_4$ requires C, 62.90; H, 7.92; N, 5.24%); m/e ($CI^+$) 268 ($M+H^+$); δ ($CDCl_3$, 360MHz) 1.44 and 1.48 (total 9H, each s, C($CH_3$)3, rotamers); 1.80-1.88 (2H, m, 4-CH and 6-CH); 2.76-2.83 (1H, m, 5-CH); 2.87-2.93 (1H, m, 3-CH); 3.00-3.09 (1H, m, 5-CH); 3.21 (1H, d, J = 10Hz, 3-CH); 3.64 and 3.66 (total 3H, each s, $C0_2CH_3$, rotamers); 4.93 and 5.13 (total 1H, each broad s, 1-CH); 6.31-6.37 and 6.40-6.45 (each 1H, each m, 7-CH and 8-CH). Isomer B is the 1R*, 6S* isomer, oil. (Found: C, 62.60; H, 7.90; N, 5.22. $C_{14}H_{21}NO_4$ requires: C, 62,90; H, 7.92; N, 5.24%); δ ($CDCl_3$, 360MHz) 1.41 and 1.43 (total 9H, each s, $CCH_3$)3, rotamers); 2.07-2.13 (1H, m, 6-CH); 2.49-2.58 (1H, m, 5-CH); 2.71-2.80 (1H, m, 4-CH) 2.88-2.97 (1H, m, 3-CH); 3.34 (1H, t, J = 12Hz, 5-CH); 3.64-3.69 (1H, m, 3-CH); 3.68 and 3.72 (total 3H, each s, $CO_2CH_3$, rotamers); 4.89 and 5.00 (total 1H, each broad s, 1-CH); 6.40-6.49 (2H, m, 7-CH and 8-CH).

b) 2-t-Butoxycarbonyl-6-[5-(3-cyclopropyl-1,2,4-oxadiazol)-yl]-2-azabicyclo[2.2.2]oct-7-ene

The foregoing ester, as a mixture of diastereomers (3.0g, 11.2mmol) was treated with cyclopropane carboxamide oxime (2.3g, 23.3mmol) and sodium hydride (1.1g of a 55% dispersion in oil, 25.2mmol) in tetrahydrofuran, exactly as described in Example 37(b). Purification of the crude product by column chromatography on silica in dichloromethane/ethyl acetate (98:2) yielded pure isomer A (993mg), (first eluted), pure isomer B (1.24g), along with mixed fractions (620mg). Isomer A, the 1R*, 6R* (anti) isomer crystallised on standing, m.p. 96-97°C (Found: C, 64.23; H, 7.34; N, 13.11. $C_{17}H_{23}N_3O_3$ requires: C, 64.33; H, 7.30; N, 13.24%); m/e ($CI^+$) 318 ($M+H^+$); δ ($CDCl_3$, 360MHz) 0.96-1.04 (4H, m, cyclopropyl $CH_2CH_2$); 1.45 and 1.49 (total 9H, each s, $C(CH_3)_3$; 1.89-1.96 (1H, m, cyclopropyl CH); 1.96-2.08 (1H, m, 4-CH); 2.13 (1H, t, J = 12Hz, 5-CH); 2.82-3.02 (2H, m, 3-CH and 5-CH); 3.30 (1H, d, J = 10Hz, 3-CH); 3.50-3.62 (1H, m, 6-CH); 5.03 and 5.24 (total 1H, each broad s, 1-CH, rotamers); 6.21-6.32 and 6.38-6.50 (each 1H, each m, 7-CH and 8-CH). Isomer B is the 1R*, 6S* isomer, oil. δ ($CDCl_3$, 360MHz) 0.98-1.04 (4H, m, cyclopropyl $CH_2CH_2$); 1.31 and 1.38 (total 9H, each s, $C(CH_3)_3$, rotamers); 1.78-1.86 (1H, m, cyclopropyl CH); 2.02-2.08 (1H, m, 4-CH); 2.22-2.28 (1H, m, 5-CH); 2.80-2.92 (1H, m, 3-CH); 2.95-2.99 (1H, m, 3-CH); 3.06-3.14 (1H, m, 3-CH); 3.45 (1H, dt, J = 1Hz and 10Hz, 6-CH); 4.81-4.84 and 4.91-4.95 (total 1H, each m, 1-CH, rotamers); 6.46-6.55 (2H, m, 7-CH and 8-CH).

c) 1R*, 6R* - 6-[5-(3-Cyclopropyl-1,2,4-oxadiazol)-yl]-2-azabicyclo[2.2.2]oct-7-ene hydrochloride

The foregoing t-butyl carbamate, isomer A, (917mg, 2.89mmol) was treated as described in Example 37(c) to yield the deprotected amine free base (573mg, 91%), which was converted to the hydrochloride salt and recrystallised from ethanol (recovery 534mg, 73%), m.p. 175-177°C. (Found : C, 56.56; H, 6.37; N, 16.49. $C_{12}H_{15}N_3O.HCl$ requires : c, 56.80; H, 6.36; N, 16.56%); m/e ($CI^+$) 218 ($M+H^+$); δ ($D_2O$, 360MHz) 0.92-0.97 and 1.09-1.16 (each 2H, each m, cyclopropyl $CH_2CH_2$); 1.83-1.91 (1H, m, 5-CH, syn to oxadiazole); 2.03-2.11 (1H, m, cyclopropyl CH); 2.38 (1H, ddd, J = 3Hz, 10Hz and 13Hz, 5-CH, anti to oxadiazole); 2.87 (1H, dt, J = 3Hz and 13Hz, 3-CH); 3.16-3.22 (1H, m, 4-CH); 3.31 (1H, dd, J = 2Hz and 13Hz, 3-CH); 3.89-3.95 (1H, m, 6-CH); 4.70-4.79 (1H, m, 1-CH, partly obscured by solvent peak); 6.32 (1H, t, J = 6Hz, 8-CH); 6.87 (1H, t, J = 6Hz, 7-CH).

d) 1R*, 6S* - 6-[5-(3-Cyclopropyl-1,2,4-oxadiazol)-yl]-2-azabicyclo[2.2.2]oct-7-ene hydrochloride

The foregoing t-butyl carbamate (1.18g, 3.72mmol) was treated as above to yield the deprotected amine free base (740mg, 92%) which was converted to the hydrochloride salt and recrystallised from ethanol (recovery 542mg) m.p. 180-181°C. (Found: C, 56.79; H, 6.34; N, 16.51. $C_{12}H_{15}N_3O.HCl$ requires: C, 56.80; H, 6.36; N, 16.56%); m/e ($CI^+$) 218 ($M+H^+$); δ ($D_2O$, 360MHz) 1.00-1.06 and 1.12-1.19 (each 2H, each m, cyclopropyl $CH_2CH_2$); 2.00-2.19 (3H, m, 5-$CH_2$ and cyclopropyl CH); 2.91 (1H, dt, J = 3Hz and 11Hz, 3-CH); 3.12-3.19 (1H, m, 4-CH); 3.33 (1H, dd, J = 2Hz and 11Hz, 3-CH); 3.48 (1H, ddd, J = 2Hz, 5Hz and 12Hz, 6-CH); 4.61-4.64 (1H, m, 1-CH); 6.56 (1H, dt, J = 1Hz and 7Hz, 8-CH); 6.86 (1H, t, J = 7Hz, 7-CH).

## EXAMPLE 41

exo-3-[5-(3-n-Butyl-1,2,4-oxadiazol)-yl]-1-azabicyclo[2.2.1]heptane

This was prepared from pentanamide oxime and 3-methoxycarbonyl-1-azabicyclo[2.2.1]heptane as described in Example 1. (Found: $M^+$, 221.1514, $C_{12}H_{19}N_3O$ requires 221.1528); δ ($CDCl_3$, 360MHz) 0.94 (3H, t, J = 7Hz, $CH_3$); 1.26-1.35 (1H, m, 5-CH); 1.39 (2H, sextet, J = 7Hz, $CH_2CH_3$); 1.67-1.76 (3H, m, 5-CH and $CH_2CH_2CH_3$); 2.44 (1H, d, J = 10Hz), 2.50-2.58 (1H, m), 2.70 (2H, t, J = 7Hz), 2.76-3.02 (5H, m) and 3.13 (1H, ddd, J = 2.5, 5, 12Hz, 2-$CH_2$, 3-CH, 4-CH, 6-$CH_2$, 7-$CH_2$ and $CH_2CH_2CH_2CH_3$).

## EXAMPLE 42

### Tablet Preparation

Tablets containing 1.0, 2.0, 25.0, 26.0, 50.0 and 100.0 mg., respectively, of the following compound is prepared as illustrated below:

3-[5-(3-cyclopropyl-1,2,4-oxadiazol)-yl]-1-azabicyclo[2.2.1]heptane.

TABLE FOR DOSES CONTAINING FROM 1-25 MG OF THE ACTIVE COMPOUND

| | Amount-mg | | |
|---|---|---|---|
| Active Compound | 1.0 | 2.0 | 25.0 |
| Microcrystalline cellulose | 49.25 | 48.75 | 37.25 |
| Modified food corn starch | 49.25 | 48.75 | 37.25 |
| Magnesium stearate | 0.50 | 0.50 | 0.50 |

TABLE FOR DOSES CONTAINING FROM 26-100 MG OF THE ACTIVE COMPOUND

| | Amount-mg | | |
|---|---|---|---|
| Active Compound | 26.0 | 50.0 | 100.0 |
| Microcrystalline cellulose | 52.0 | 100.0 | 200.0 |
| Modified food corn starch | 2.21 | 4.25 | 8.5 |
| Magnesium stearate | 0.39 | 0.75 | 1.5 |

All of the active compound, lactose, and a portion of the corn starch are mixed and granulated to a 10% corn starch paste. The resulting granulation is sieved, dried and blended with the remainder of the corn starch and the magnesium stearate. The resulting granulation is then compressed into tablets containing 1.0 mg, 2.0 mg, 25.0 mg, 26.0 mg, 50.0 mg, and 100.0 mg of active ingredient per tablet.

**Claims**

1. A compound of formula I, or a salt or prodrug thereof:

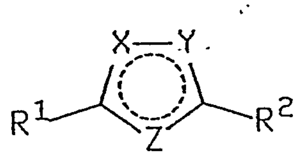

(I)

wherein one of X, Y and Z is an oxygen atom and the other two are nitrogen atoms, and the dotted circle represents aromaticity (two double bonds) thus forming a 1,3,4-oxadiazole or 1,2,4-oxadiazole nucleus; $R^1$ represents a non-aromatic azacyclic or azabicyclic ring system; and $R^2$ represents an optionally substituted saturated hydrocarbon group having at least three carbon atoms, or unsaturated hydrocarbon group having at least 6 carbon atoms.

2. A compound as claimed in claim 1 wherein $R^1$ represents pyrrolidine, piperidine, tetrahydropyridine, 1- or 2-azanorbornane, quinuclidine, isoquinuclidine or 2-azabicyclo[2.2.2]octene, any of which may be

optionally substituted with methyl or hydroxy.

3. A compound as claimed in claim 1 or claim 2 wherein $R^2$ represents n-propyl, isopropyl, cyclopropyl, n-butyl, benzyl, phenylethenyl or pyridylmethyl, optionally substituted by one or more substituents selected from methyl, ethyl, vinyl, phenyl, naphthyl, pyridyl, acetoxy, keto, fluoro, hydroxy and methoxy.

4. A compound as claimed in any one of the preceding claims wherein $R^2$ represents a group of structure:

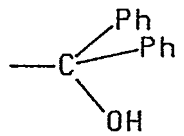

5. A compound as claimed in claim 1 of formula II:

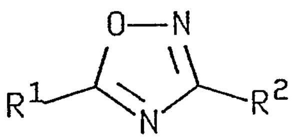

(II)

wherein $R^1$ and $R^2$ are as defined in claim 1.

6. A compound as claimed in claim 1 selected from:
3-[5-(3-benzyl-1,2,4-oxadiazol)yl]quinuclidine;
3-[5-(3-cyclopropyl-1,2,4-oxadiazol)yl]quinuclidine;
3-[5-(3-(1-phenyl)ethyl-1,2,4-oxadiazol)yl]quinuclidine;
3-[5-(3-(1-adamantyl)-1,2,4-oxadiazol)yl]quinuclidine;
3-[5-(3-cyclopropyl-1,2,4-oxadiazol)yl]-1-azabicyclo[2.2.1]heptane;
3-[5-(3-cyclopropyl-1,2,4-oxadiazol)yl]-1,2,5,6-tetrahydropyridine;
3-[5-(3-benzyl-1,2,4-oxadiazol)yl]-1,2,5,6-tetrahydropyridine;
3-[5-(3-isopropyl-1,2,4-oxadiazol)yl]-1,2,5,6-tetrahydropyridine;
3-cyclopropyl-5-(5-ethyl-1,2,5,6-tetrahydropyrid-3-yl)-1,2,4-oxadiazole;
3-isopropyl-5-(5-ethyl-1,2,5,6-tetrahydropyrid-3-yl)-1,2,4-oxadiazole;
3-cyclopropyl-5-(5-methyl-1,2,5,6-tetrahydropyrid-3-yl)-1,2,4-oxadiazole;
3-isopropyl-5-(5-methyl-1,2,5,6-tetrahydropyrid-3-yl)-1,2,4-oxadiazole;
3-[3-(1-hydroxy-1-phenylmethyl)-1,2,4-oxadiazol-5-yl]quinuclidine;
3-[3-(1-acetoxy-1-phenylmethyl)-1,2,4-oxadiazol-5-yl]quinuclidine;
3-(3-benzoyl-1,2,4-oxadiazol-5-yl)quinuclidine;
3-[3-(1-hydroxy-1-phenylethyl)-1,2,4-oxadiazol-5-yl]quinuclidine;
3-[3-(1,1-diphenyl-1-hydroxymethyl)-1,2,4-oxadiazol-5-yl]quinuclidine;
3-[3-(2-methoxy-1-phenylethenyl)-1,2,4-oxadiazol-5-yl]quinuclidine;
3-[5-(1-hydroxy-1-phenylmethyl)-1,2,4-oxadiazol-3-yl]quinuclidine;
3-[3-(1,1-diphenyl-1-hydroxymethyl)-1,2,4-oxadiazol-5-yl]-1,2,5,6-tetrahydropyridine;
3-[3-(1-hydroxy-1-phenylmethyl)-1,2,4-oxadiazol-5-yl]-1,2,5,6-tetrahydropyridine;
3-[3-(4-chlorophenyl)-1,2,4-oxadiazol-5-yl]-1,2,5,6-tetrahydropyridine;
5-hydroxy-3-(3-isopropyl-1,2,4-oxadiazol-5-yl)-1-azabicyclo[2.2.l]heptane;
3-(3-cyclopropyl-1,2,4-oxadiazol-5-yl)-5-hydroxy-1-azabicyclo[2.2.1]heptane;
5-hydroxy-3-(3-n-propyl-1,2,4-oxadiazol-5-yl)-1-azabicyclo[2.2.1]heptane;
3-(3-n-propyl-1,2,4-oxadiazol-5-yl)-1-azabicyclo[2.2.1]heptane;
3-(3-cyclopropyl-1,2,4-oxadiazol-5-yl)-1-azabicyclo[2.2.1]heptane;
3-(3-isopropyl-1,2,4-oxadiazol-5-yl)-1-azabicyclo[2.2.1]heptane;
3-[3-(1-phenylethyl)-1,2,4-oxadiazol-5-yl]-1-azabicyclo[2.2.1]heptane;
3-[3-(1-hydroxy-1-phenylpropyl)-1,2,4-oxadiazol-5-yl]quinuclidine;
3-[3-(1,1-diphenylmethyl)-1,2,4-oxadiazol-5-yl]quinuclidine;
3-[3-(1-hydroxy-1-phenylprop-2-enyl)-1,2,4-oxadiazol-5-yl]quinuclidine;
3-[3-(1-hydroxy-1-(2-naphthyl)-1-phenylmethyl)-1,2,4-oxadiazol-5-yl]quinuclidine;
3-[3-(1-(4-fluorophenyl)-1-hydroxy-1-phenylmethyl)-1,2,4-oxadiazol-5-yl]quinuclidine;
3-[3-(1-hydroxy-1-(4-methoxyphenyl)-1-phenylmethyl)-1,2,4-oxadiazol-5-yl]quinuclidine;

3-[3-(1,1-diphenyl-1-hydroxymethyl)-1,2,4-oxadiazol-5-yl]-1-azabicyclo[2.2.1]heptane;
3-[3-(1-hydroxy-1-(pyrid-2-yl)methyl-1,2,4-oxadiazol-5-yl]quinuclidine;
3-[3-(1-hydroxy-1-phenyl-1-(pyrid-2-yl)methyl-1,2,4-oxadiazol-5-yl]quinuclidine;
6-(3-cyclopropyl-1,2,4-oxadiazol-5-yl)-2-azabicyclo[2.2.2]octane;
6-(3-isopropyl-1,2,4-oxadiazol-5-yl)-2-azabicyclo[2.2.2]octane;
6-(3-cyclopropyl-1,2,4-oxadiazol-5-yl)-2-methyl-2-azabicyclo[2.2.2]octane;
6-(3-cyclopropyl-1,2,4-oxadiazol-5-yl)-2-azabicyclo[2.2.2]oct-7-ene;
3-(3-n-butyl-1,2,4-oxadiazol-5-yl)-1-azabicyclo[2.2.1]heptane;
and salts and prodrugs thereof.

7. A pharmaceutical composition comprising a compound as claimed in any one of the preceding claims in association with a pharmaceutically acceptable carrier or excipient.

8. A compound as claimed in any one of claims 1 to 6 for use as a therapeutic agent.

9. The use of a compound as claimed in any one of claims 1 to 6 for the preparation of a medicament for the treatment and/or prevention of neurological and mental disorders.

10. A process for the preparation of a compound as claimed in any one of claims 1 to 6 which comprises reacting a reactive derivative of a carboxylic acid of formula $R^a$-$CO_2H$ with a compound either of formula IIIA or of formula IIIB, or a salt thereof:

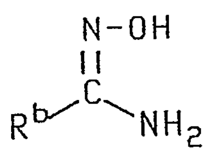

(IIIA)

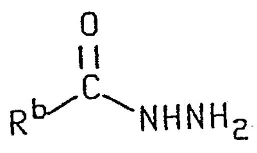

(IIIB)

wherein one of $R^a$ and $R^b$ is a non-aromatic azacyclic or azabicyclic ring, and the other is an optionally substituted saturated hydrocarbon group having at least three carbon atoms, or unsaturated hydrocarbon group having at least 6 carbon atoms.

**Claims for the following Contracting States: ES, GR**

1. A process for the preparation of a compound of formula I, or a salt or prodrug thereof:

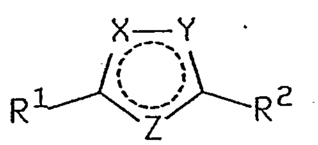

(I)

wherein one of X, Y and Z is an oxygen atom and the other two are nitrogen atoms, and the dotted circle represents aromaticity (two double bonds) thus forming a 1,3,4-oxadiazole or 1,2,4-oxadiazole nucleus; $R^1$ represents a non-aromatic azacyclic or azabicyclic ring system; and $R^2$ represents an optionally substituted saturated hydrocarbon group having at least three carbon atoms, or unsaturated hydrocarbon group having at least 6 carbon atoms; which process comprises reacting a reactive derivative of a carboxylic acid of formula $R^a$-$CO_2H$ with a compound either of formula IIIA or of formula IIIB, or salt thereof:

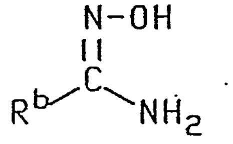

(IIIA)

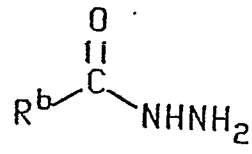

(IIIB)

wherein one of $R^a$ and $R^b$ is a non-aromatic azacyclic or azabicyclic ring, and the other is an optionally substituted saturated hydrocarbon group having at least three carbon atoms, or unsaturated hydrocarbon group having at least 6 carbon atoms.

2. A process as claimed in claim 1 for the preparation of a compound wherein $R^1$ represents pyrrolidine, piperidine, tetrahydropyridine, 1- or 2-azanorbornane, quinuclidine, isoquinuclidine or 2-azabicyclo[2.2.2]octene, any of which may be optionally substituted with methyl or hydroxy.

3. A process as claimed in claim 1 or claim 2 for the preparation of a compound wherein $R^2$ represents n-propyl, isopropyl, cyclopropyl, n-butyl, benzyl, phenylethyl or pyridylmethyl, optionally substituted by one or more substituents selected from methyl, ethyl, vinyl, phenyl, naphthyl, pyridyl, acetoxy, keto, fluoro, hydroxy and methoxy.

4. A process as claimed in any one of the preceding claims for the preparation of a compound wherein $R^2$ represents a group of structure:

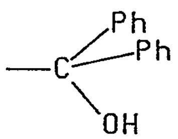

5. A process as claimed in claim 1 for the preparation of a compound of formula II:

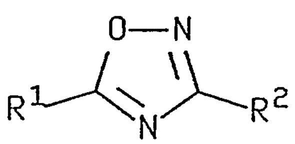

(II)

wherein $R^1$ and $R^2$ are as defined in claim 1.

6. A process as claimed in claim 1 for the preparation of a compound selected from:
3-[5-(3-benzyl-1,2,4-oxadiazol)yl]quinuclidine;
3-[5-(3-cyclopropyl-1,2,4-oxadiazol)yl]quinuclidine;
3-[5-(3-(1-phenyl)ethyl-1,2,4-oxadiazol)yl]quinuclidine;
3-[5-(3-(1-adamantyl)-1,2,4-oxadiazol)yl]quinuclidine;
3-[5-(3-cyclopropyl-1,2,4-oxadiazol)yl]-1-azabicyclo[2.2.1]heptane;
3-[5-(3-cyclopropyl-1,2,4-oxadiazol)yl]-1,2,5,6-tetrahydropyridine;
3-[5-(3-benzyl-1,2,4-oxadiazol)yl]-1,2,5,6-tetrahydropyridine;
3-[5-(3-isopropyl-1,2,4-oxadiazol)yl]-1,2,5,6-tetrahydropyridine;
3-cyclopropyl-5-(5-ethyl-1,2,5,6-tetrahydropyrid-3-yl)-1,2,4-oxadiazole;
3-isopropyl-5-(5-ethyl-1,2,5,6-tetrahydropyrid-3-yl)-1,2,4-oxadiazole;
3-cyclopropyl-5-(5-methyl-1,2,5,6-tetrahydropyrid-3-yl)-1,2,4-oxadiazole;
3-isopropyl-5-(5-methyl-1,2,5,6-tetrahydropyrid-3-yl)-1,2,4-oxadiazole;
3-[3-(1-hydroxy-1-phenylmethyl)-1,2,4-oxadiazol-5-yl]quinuclidine;
3-[3-(1-acetoxy-1-phenylmethyl)-1,2,4-oxadiazol-5-yl]quinuclidine;
3-(3-benzoyl-1,2,4-oxadiazol-5-yl)quinuclidine;
3-[3-(1-hydroxy-1-phenylethyl)-1,2,4-oxadiazol-5-yl]quinuclidine;

3-[3-(1,1-diphenyl-1-hydroxymethyl)-1,2,4-oxadiazol-5-yl]quinuclidine;
3-[3-(2-methoxy-1-phenylethenyl)-1,2,4-oxadiazol-5-yl]quinuclidine;
3-[5-(1-hydroxy-1-phenylmethyl)-1,2,4-oxadiazol-3-yl]quinuclidine;
3-[3-(1,1-diphenyl-1-hydroxymethyl)-1,2,4-oxadiazol-5-yl]-1,2,5,6-tetrahydropyridine;
3-[3-(1-hydroxy-1-phenylmethyl)-1,2,4-oxadiazol-5-yl]-1,2,5,6-tetrahydropyridine;
3-[3-(4-chlorophenyl)-1,2,4-oxadiazol-5-yl]-1,2,5,6-tetrahydropyridine;
5-hydroxy-3-(3-isopropyl-1,2,4-oxadiazol-5-yl)-1-azabicyclo[2.2.1]heptane;
3-(3-cyclopropyl-1,2,4-oxadiazol-5-yl)-5-hydroxy-1-azabicyclo[2.2.1]heptane;
5-hydroxy-3-(3-n-propyl-1,2,4-oxadiazol-5-yl)-1-azabicyclo[2.2.1]heptane;
3-(3-n-propyl-1,2,4-oxadiazol-5-yl)-1-azabicyclo[2.2.1]heptane;
3-(3-cyclopropyl-1,2,4-oxadiazol-5-yl)-1-azabicyclo[2.2.1]heptane;
3-(3-isopropyl-1,2,4-oxadiazol-5-yl)-1-azabicyclo[2.2.1]heptane;
3-[3-(1-phenylethyl)-1,2,4-oxadiazol-5-yl]-1-azabicyclo[2.2.1]heptane;
3-[3-(1-hydroxy-1-phenylpropyl)-1,2,4-oxadiazol-5-yl]quinuclidine;
3-[3-(1,1-diphenylmethyl)-1,2,4-oxadiazol-5-yl]quinuclidine;
3-[3-(1-hydroxy-1-phenylprop-2-enyl)-1,2,4-oxadiazol-5-yl]quinuclidine;
3-[3-(1-hydroxy-1-(2-naphthyl)-1-phenylmethyl)-1,2,4-oxadiazol-5-yl]quinuclidine;
3-[3-(1-(4-fluorophenyl)-1-hydroxy-1-phenylmethyl)-1,2,4-oxadiazol-5-yl]quinuclidine;
3-[3-(1-hydroxy-1-(4-methoxyphenyl)-1-phenylmethyl)-1,3,4-oxadiazol-5-yl]quinuclidine;
3-[3-(1,1-diphenyl-1-hydroxymethyl)-1,2,4-oxadiazol-5-yl]-1-azabicyclo[2.2.1]heptane;
3-[3-(1-hydroxy-1-(pyrid-2-yl)methyl-1,2,4-oxadiazol-5-yl]quinuclidine;
3-[3-(1-hydroxy-1-phenyl-1-(pyrid-2-yl)methyl-1,2,4-oxadiazol-5-yl]quinuclidine;
6-(3-cyclopropyl-1,2,4-oxadiazol-5-yl)-2-azabicyclo[2.2.2]octane;
6-(3-isopropyl-1,2,4-oxadiazol-5-yl)-2-azabicyclo[2.2.2]octane;
6-(3-cyclopropyl-1,2,4-oxadiazol-5-yl)-2-methyl-2-azabicyclo[2.2.2]octane;
6-(3-cyclopropyl-1,2,4-oxadiazol-5-yl)-2-azabicyclo[2.2.2]oct-7-ene;
3-(3-n-butyl-1,2,4-oxadiazol-5-yl)-1-azabicyclo[2.2.1]heptane;
and salts and prodrugs thereof.

7. A process for the preparation of a pharmaceutical composition which comprises mixing a compound prepared as claimed in any one of the preceding claims with a pharmaceutically acceptable carrier or excipient.

8. The use of a compound prepared as claimed in any one of claims 1 to 6 for the preparation of a medicament for the treatment and/or prevention of neurological and mental disorders.